# EUROPEAN PATENT APPLICATION

(11) **EP 2 270 126 A1**
(43) Date of publication of application: **05.01.2011**
(21) Application number: 09732387.7
(22) Date of filing: 15.04.2009
(51) Int. Cl.: C12M 1/00, C12M 1/42, C12Q 1/04, G01N 33/53

(54) **CELL SELECTION APPARATUS, AND CELL SELECTION METHOD USING THE SAME**

(30) Priority: 15.04.2008 JP 2008105396
(71) Applicant: Tosoh Corporation, Yamaguchi 746-8501 (JP)
(72) Inventor: YAMANAKA, Maho, Ayase-shi Kanagawa 252-1123 (JP); MARUYAMA, Takahiro, Ayase-shi Kanagawa 252-1123 (JP); FUTAMI, Toru, Ayase-shi Kanagawa 252-1123 (JP)
(74) Representative: Novagraaf Technologies
(86) International application number: PCT/JP2009/057611
(87) International publication number: WO 2009/128483

(57) **Abstract**

Disclosed are a cell selection apparatus and a cell selection method for selecting a mass of cells capable of producing a specific substance, all at once easily in a short period of time, and further, for efficiently selecting these cells based on the strength of a binding force between the specific substance on the surface of the cell and a recognition molecule. The cell selection apparatus includes: a cell selection vessel which has a pair of electrodes and a sheet-like insulating material having a plurality of micropores, with a recognition molecule bindable to the specific substance being disposed on a bottom face of the micropore; and a power supply, wherein the power supply includes a cell-immobilization power supply and a cell-taking power supply. The cell selection method uses the cell selection apparatus, including; introducing cells into a cell selection area; immobilizing these cells in the micropores; effecting a binding reaction between the specific substance on the surface of the cell and the recognition molecule; thereafter, taking out a cell of which the specific substance on the surface of the cell is not or is weakly bound to the recognition molecule, among the entire population of the cells, from the micropore; otherwise alternatively, leaving a cell of which the specific substance on the surface of the cell is strongly bound to the recognition molecule, among the entire population of the cells, behind in the micropore.

## Description

### TECHNICAL FIELD

The present invention relates to a cell selection apparatus for efficiently selecting cells, and a cell selection method using the same.
Priority is claimed on Japanese Patent Application No. 2008-105396, filed April 15, 2008, the content of which is incorporated herein by reference.

### BACKGROUND ART

In recent years, the use of antibodies produced in vivo is attracting an attention as raw materials of diagnostic agents and drugs for diseases. Antibodies are a kind of protein which specifically binds to a foreign matter such as a virus when the foreign matter enters an organism body. An antibody has a function to specifically bind to a specific foreign matter such as a virus to thereby kill or detoxify the virus so as to protect an organism from the invasion of the virus. Here, the foreign matter such as a virus which specifically binds with an antibody is generally called an antigen. Moreover, antibodies in vivo are produced from cells residing in the spleen or lymph nodes. It is said that there are more than a million kinds of antibodies. The cells which produce antibodies in the spleen or lymph nodes are called lymphocytes, B cells, antibody-producing cells, or such names.

In addition, the above-mentioned diagnostic agents and drugs make use of this "property to specifically and strongly bind to a specific substance" of antibodies. Particularly, diagnostic agents using antibodies are called immunodiagnostic agents, and drugs using antibodies are called antibody drugs, or such names.

Immunodiagnostic agents can be exemplified by those used for the diagnosis of infectious diseases through detection of a virus or such foreign matter which has entered an organism body, by using an antibody, or those used for the diagnosis of cancer, heart disease, autoimmune disease, or such a disease through detection of a protein or like substance that is highly correlated with the disease. In particular, immunodiagnostic agents are widely used in the field of clinical diagnosis as highly sensitive and highly reliable diagnostic agents because antibodies are specifically bindable to specific substances.

Moreover, antibody drugs can be exemplified by drugs which kill pathogenic microbes which have entered an organism body, drugs which detoxify disease-inducing proteins, drugs which inhibit the proliferation of cancer cells, drugs which kill cancer cells, or drugs which suppress allergic reactions. In particular, antibody drugs are expected as drugs with fewer side-effects because antibodies are specifically bindable to specific substances.

Usually, antibodies for use in an immunodiagnostic agent or an antibody drug are composed of only a single kind of antibodies, which are generally called monoclonal antibodies.

However, antibody-producing cells are not able to proliferate when taken out from the organism body, and it is not possible with these cells per se to produce a mass of antibodies on an industrial basis. Therefore, methods for industrial mass production of antibodies are employed in which an antibody-producing cell taken out from the organism body and an infinite proliferative cell such as a cancer cell are fused to thereby generate a cell which can infinitely proliferate and can produce antibodies (hereunder, referred to as a fused cell or a hybridoma). The following steps 1) to 3) show representative examples to generate a fused cell for the industrial mass production of monoclonal antibodies.
1) An antigen of interest serving as a foreign matter to be detected or to be treated is injected into an animal such as a mouse or a rat, to thereby make the animal produce antibodies specifically bindable to the antigen inside its body (hereunder, this process is referred to as immunization).
2) Antibody-producing cells are taken out from the spleen or a lymph node of the immunized animal of 1), and contacted and fused with cancer cells within a medium such as polyethylene glycol which improves the fluidity of cell membranes (PEG method). Or, otherwise, antibody-producing cells and cancer cells in a solvent located between electrodes are applied with an AC voltage to effect a contact between these cells, and thereafter applied with a DC pulse voltage to effect a temporal breakage of cell membranes of both cells which are in contact with each other, followed by a membrane regeneration to thereby effect a fusion between them (electrofusion method) (hereunder, this process is referred to as cell fusion).
3) Among these fused cells generated from 2), cells particularly capable of producing antibodies which are specifically and strongly bindable to the antigen of interest are selected (hereunder, this process is referred to as cell selection).

Generally speaking, antibody-producing cells are taken out from the spleen of an immunized mouse (hereunder, referred to as spleen cells). The number of these cells is about one to two hundred million. Of these, the number of fused cells generated by cell fusion is about several thousand. Furthermore, of these fused cells, the number of fused cells capable of producing antibodies which are specifically and strongly bindable to the antigen of interest is only a few. Accordingly, it is necessary to efficiently examine the entire population of several thousand of fused cells, regarding whether or not these fused cells are capable of producing antibodies which are specifically and strongly bindable to the antigen of interest, through detection of antibodies produced by these cells in the step 3) of cell selection mentioned above.

As a method for detecting antibodies produced by cells, generally used is an immunochemical assay method. This is a method for detecting an antibody based on an antigen-antibody reaction in which an antibody specifically recognizes an antigen, and this method is recently drawing attention because of its excellent accuracy, convenience, rapidity, and inexpensiveness. In the immunochemical assay method, quite a large variety of labels, for example, an enzyme, a radiotracer, chemiluminescent and fluorescent labels, metallic atom and sol, stable free radicals, latex, and a bacteriophage, have been applied as a detection method.

Of the immunochemical assay methods, an Enzyme-Linked ImmunoSorbent (adsorption) Assay method (hereunder, referred to as an ELISA method) which uses an enzyme is now widely used as a particularly excellent method in terms of inexpensiveness and convenience. The ELISA method uses, for example, a 96 well-microtiter plate made of polystyrene, as a support to immobilize antigens or antibodies. After the antigen or antibody has been immobilized in each well, an antigen or antibody labeled by an indirect competitive method, a direct competitive method, or such a method is detected to thereby detect the quantity of the analyte substance. The conventional ELISA method is known as an easy and quick assay method, but it is supposed to require about several hours for the measurement. Moreover, the number of samples that can be detected all at once is limited to the number of wells per plate. Accordingly, there has been a demand for an easy and quick assay method which can measure a mass of samples in a shorter period of time than that of the conventional ELISA method.

In contrast, in order to solve the problems involved in the ELISA method mentioned above, an example of a detection method has been reported in which: a substance (for example, an antigen) that can specifically react with an analyte substance (for example, an antibody) is immobilized on a porous material (hereunder, referred to as an immobilized substance); a solution containing the analyte substance is filtered by the porous material, thus allowing the analyte substance to react with the immobilized substance on the porous material; then a labeled substance that can react with the immobilized substance competitively with the analyte substance is filtered by the porous material, thus allowing the labeled substance to react with the immobilized substance; and the quantity of the analyte substance is detected by measuring the quantity of the labeled substance which reacts with the immobilized substance (for example, refer to Patent Document 1).

The method described in Patent Document 1 is a detection method for an analyte substance using an antigen-antibody reaction. In this method, it is supposed to be preferable to perform the filtration with the porous material by using a plate including a plurality of wells consisting of wall portions and bottom portions, and to arrange the porous material on the bottom portions of the wells. Such a plate is commercially available under the product name of "MultiScreen (registered trademark)" from Millipore Corporation. Known plates are the "IP" filter plate using hydrophobic PVDF (polyvinylidene fluoride) as a porous material, the "HA" filter plate using mixed cellulose esters as a porous material, the "NC" filter plate using 100% nitrocellulose as a porous material, and the like. These plates have 96 wells.

The method described in Patent Document 1 is capable of providing excellent detection sensitivity within a short period of time of about 30 minutes, by filtering the analyte solution and then filtering the labeled substance that can react with the immobilized substance competitively with the analyte substance. However, the method described in Patent Document 1 performs the filtration by using a plate having a plurality of wells consisting of wall portions and bottom portions mentioned above, involving a problem in that it is difficult to assay a mass of samples all at once. Here, generally and often used is a 96 well-plate as mentioned above.

On the other hand, there is a method in which an antibody-producing cell capable of producing an antibody of interest is selected before the production of a fused cell, and then the selected antibody-producing cell is used for cell fusion. As a technique for selecting and collecting this antibody-producing cell capable of producing an antibody of interest, a collection technique by means of a magnetic bead system or a collection technique utilizing flow cytometry are widely applied at present.
In the magnetic bead system, using magnetic beads the surfaces of which are bound with monoclonal antibodies capable of specifically reacting with a specific substance presented on the surface of the target cell, target cells are selectively bound to the magnetic beads through an antigen-antibody reaction and these beads are selectively collected through a magnetic column (hereunder, referred to as a magnetic bead method). This method has an advantage in that a mass of, about 10⁹, target cells can be selected with a recovery rate of about 90% all at once (for example, refer to Patent Document 2 and Non-Patent Document 1). However, with this method, sometimes cases arise in which the target cells have to be separated from the magnetic beads bound through the antigen-antibody reaction, involving a problem of complicating its process. Furthermore, with this method, cells are selected based on whether or not these cells are bound through the antigen-antibody reaction, so it is not possible to select cells based on the strength of the binding force in the antigen-antibody reaction.

On the other hand, flow cytometry is a method to identify cells based on fluorescence emitting from antibodies after binding fluorescently labeled monoclonal antibodies to a specific substance presented on the surface of the target cell through an antigen-antibody reaction (hereunder, referred to as a flow cytometry method). Specifically speaking, cells are suspended in a jet stream ejecting from a nozzle, then ultrasonic vibration is applied to this jet stream so that the stream breaks into droplets, and electric charges are applied to the water stream just before the stream breaks into droplets so that droplets containing the target cells (that is, fluorescently labeled cells) are negatively or positively charged. Then, these droplets are allowed to fall through an electric field that diverts charged droplets, and thereby the target cells are collected. This method yields a higher purity than that of the magnetic bead system, and is capable of detecting about six different types of cells at the same time by using a combination of a plurality of laser beams and a plurality of fluorescent wavelengths (for example, refer to Patent Document 2). However, this method requires an expensive and large-scaled apparatus, involving a problem in that an easy and quick assay is difficult. Furthermore, with this method, cells are selected based on whether or not these cells are bound through the antigen-antibody reaction, so it is not possible to select cells based on the strength of the binding force in the antigen-antibody reaction.

In addition, as to the method in which a spleen cell capable of producing an antibody of interest is selected before the production of a fused cell, and then the selected spleen cell is used for cell fusion, there are: a method in which one spleen cell capable of specifically reacting with a particular antigen is selected, the selected spleen cell is cultured, and spleen cells proliferated by culture are fused with cancer cells to thereby produce fused cells (for example, refer to Patent Document 3); and a method in which spleen cells obtained by immunization with an antigen of interest are mixed with cancer cells on the surfaces of which the antigen of interest is presented, then, among these spleen cells, some spleen cells which present antibodies specifically bindable to the antigen of interest are selectively brought closer to the cancer cells through the specific bond with the antigen of interest on the surface of the cancer cell, and thereby both cells are fused (for example, refer to Patent Document 4).

However, with any one of these methods, the number of cells drastically decreases because the cell selection is carried out before the cell fusion. For example, the proportion of mouse spleen cells capable of producing the antibody of interest is said to be less than about 1% (about 1 × 10⁶ cells) among the entire population of the spleen cells (generally, about 1 × 10⁸ cells). Accordingly, since the cell fusion method to be conducted after the cell selection is the PEG method (for example, refer to Non-Patent Document 2 and Non-Patent Document 3) or the electrofusion method (for example, refer to Non-Patent Document 3) which are conventional cell fusion methods, the refusion probability thereof is very low at about 0.2/10000 in general, involving a problem in that it is substantially difficult to generate fused cells. In addition, the operations of the cell selection and the cell fusion are conducted in separate vessels or such separate places, involving a problem in that cells are highly likely to be lost during the replacement of vessels. Here, the term refusion probability generally means a value obtained by dividing the number of fused cells generated from the cell fusion by the number of spleen cells used for the cell fusion.

Moreover, there is also reported an example of a cell separation system capable of selecting and collecting masses of a plurality of types of cells all at once (for example, refer to Patent Document 2).

The method described in Patent Document 2 is a method in which, using a cell separation kit including a temperature-sensitive support exhibiting a hydrophilic property at a specific temperature (transition temperature) or above and exhibiting a hydrophobic property at a temperature not higher than the specific temperature, and a collecting cell attached to the surface of the support and bound with a substance having an antigen-binding structure to a target cell; the cell separation kit is mixed into an aqueous solution containing the target cell and adjusted to a temperature not lower than the transition temperature and the target cell is bound to the collecting cell through the antigen antibody reaction; a support attached with the collecting cell bound to the target cell is separated from the aqueous solution and transferred into another medium; the temperature of this medium is lowered to the transition temperature or below of the support, and the medium is attached to the support; the collecting cell bound to the target cell is taken out from the support; the support is separated from the collecting cell bound to the target cell; and a conjugate of the target cell with the collecting cell is released through pH adjustment or the like, to thereby separate the target cell from the collecting cell. This method is capable of separating masses of cells using a temperature-sensitive polymer compound by combination between the temperature-sensitive polymer compound and the collecting cell bound to a substance which can cause an antigen-antibody reaction with the target cell. However, there is a problem in that the process for separating target cells from collecting cells is complicated. Furthermore, with this method, cells are selected based on whether or not these cells are bound through the antigen-antibody reaction, so it is not possible to select cells based on the strength of the binding force in the antigen-antibody reaction.

### PRIOR ART DOCUMENTS

### PATENT DOCUMENTS

Patent Document 1: Japanese Laid-Open Patent Application No. 2001-281250
Patent Document 2: Japanese Laid-Open Patent Application No. 2004-73112
Patent Document 3: Japanese Patent No. 3799392
Patent Document 4: Japanese Laid-Open Patent Application No. 2006-6250

### NON-PATENT DOCUMENTS

Non-Patent Document 1: Hiroki Murakami and Takuya Sugawara: Saibou Kougaku Gairon (Cell Technology Compendium), First Edition, Corona Publishing Co. Ltd., pp. 181-198, September 10 (2001)
Non-Patent Document 2: Kohler G. and Milstein C., Nature, 256, pp. 495-497 (1975)
Non-Patent Document 3: De St. Groth S. F. and Schedegger D., J. Immunol. Methods, 35, pp. 1-21 (1980)
Non-Patent Document 4: U. Zimmermann, G. Pilwat, and H. Pohl, J. Biol. Phys., Volume 10, pp. 43-50 (1982)

### DISCLOSURE OF INVENTION

### PROBLEMS TO BE SOLVED BY THE INVENTION

The present invention has been proposed with consideration of such conventional situations, with an object of providing a cell selection apparatus and a cell selection method for easily selecting a mass of cells capable of producing a specific substance, all at once in a short period of time, and further, for efficiently selecting these cells based on the strength of a binding force between the specific substance on a cell surface and a recognition molecule.

### MEANS TO SOLVE THE PROBLEMS

The inventors of the present invention made an attempt to solve the abovementioned problems in the conventional techniques and to achieve the abovementioned object. As a result, they discovered that the abovementioned problems in the conventional techniques can be solved by using a cell selection apparatus including: a cell selection vessel which has a pair of electrodes formed from oppositely disposed conductive members and a sheet-like insulating material disposed between the pair of electrodes via a sheet-like spacer and having a plurality of micropores piercing in the direction of the oppositely disposed electrodes, with the insulating material being disposed on a surface of either one of the electrodes, and a recognition molecule bindable to a specific substance being disposed on a bottom face of the micropore; and a power supply for applying a voltage to the pair of electrodes, wherein the power supply includes a cell-immobilization power supply, which serves as a first AC power supply for applying a first AC voltage, and a cell-taking power supply, which serves as a second AC power supply for applying a second AC voltage, and a cell selection method using the cell selection apparatus, including; introducing cells into a cell selection area of the cell selection vessel, which serves as a space for selecting cells and is formed between the pair of electrodes via the spacer; immobilizing these cells in the micropores by applying the first AC voltage from the first AC power supply; effecting a binding reaction between the specific substance on a surface of the cell and the recognition molecule; thereafter, taking out a cell on the surface of which the specific substance is not or is weakly bound to the recognition molecule, among the entire population of the cells, from the micropore, under a specific force acting in a direction to take out the cell from the micropore; otherwise alternatively, leaving a cell on the surface of which the specific substance is strongly bound to the recognition molecule, among the entire population of the cells, behind in the micropore, under a specific force acting in a direction to take out the cell from the micropore. This finally led to the completion of the present invention.
Hereunder is a detailed description of the present invention.

The cell selection apparatus of the present invention is a cell selection apparatus including: a cell selection vessel which has a pair of electrodes formed from oppositely disposed conductive members and a sheet-like insulating material disposed between the pair of electrodes via a sheet-like spacer and having a plurality of micropores piercing in the direction of the oppositely disposed electrodes, with the insulating material being disposed on a surface of either one of the electrodes, and a recognition molecule bindable to a specific substance being disposed on a bottom face of the micropore; and a power supply for applying a voltage to the pair of electrodes, wherein the power supply includes a cell-immobilization power supply and a cell-taking power supply.

The cell selection apparatus of the present invention is also a cell selection apparatus, wherein , in the power supply of the above-mentioned cell selection apparatus, the cell-immobilization power supply includes a first AC power supply for applying a first AC voltage, the cell-taking power supply includes a second AC power supply for applying a second AC voltage, and the power supply includes a power supply-switchover mechanism for switching over between the first AC power supply and the second AC power supply.

The cell selection apparatus of the present invention is also a cell selection apparatus according to claim 3, wherein the above-mentioned power supply includes a cell-immobilization power supply, a cell-taking power supply, and a cell-fusion power supply, and the power supply has the cell-immobilization power supply including a first AC power supply for applying a first AC voltage, the cell-taking power supply including a second AC power supply for applying a second AC voltage, the cell-fusion power supply including a DC pulse power supply for applying a DC pulse voltage, and a power supply-switchover mechanism which picks up any one or two of the first AC power supply, the second AC power supply, and the DC pulse power supply, and is connected to the picked up one.

The cell selection apparatus of the present invention is also the cell selection apparatus mentioned above, wherein an AC frequency of the first AC power supply is 30 kHz or higher, and an AC frequency of the second AC power supply is lower than 20 kHz.
The cell selection apparatus of the present invention is also the cell selection apparatus mentioned above, wherein the recognition molecule bindable to the specific substance is an antigen.

The cell selection apparatus of the present invention is also the cell selection apparatus mentioned above, wherein a cationic polymer is provided on a surface of the electrode which faces the cell selection area serving as a space for selecting cells and being formed between the pair of electrodes via the spacer, and/or a surface of the micropored plate-like insulating material disposed on the electrode on the side of the insulating material, of the cell selection vessel, with the cationic polymer being a polylysine, a polylysine derivative, or an amino group-containing polymer.

The cell selection method of the present invention is a cell selection method using the above-mentioned cell selection apparatus, including; introducing cells into the cell selection area; immobilizing these cells in the micropores by applying the first AC voltage from the first AC power supply; effecting a binding reaction between the specific substance on a surface of the cell and the recognition molecule disposed on the bottom face of the micropore; and thereafter, taking out a cell of which the specific substance on the surface of the cell is not or is weakly bound to the recognition molecule, among the entire population of the cells, from the micropore, under a specific force acting in a direction to take out the cell from the micropore; otherwise alternatively, leaving a cell of which the specific substance on the surface of the cell is strongly bound to the recognition molecule, among the entire population of the cells, behind in the micropore, under a specific force acting in a direction to take out the cell from the micropore.

In addition, the cell selection method of the present invention is also a cell selection method using the above-mentioned cell selection apparatus, including the steps of; introducing first cells into the cell selection area; immobilizing these first cells in the micropores by applying the first AC voltage from the first AC power supply; effecting a binding reaction between the specific substance on a surface of the first cell and the recognition molecule; taking out a cell of which the specific substance on the surface of the cell is not or is weakly bound to the recognition molecule, among the entire population of the first cells, from the micropore, under a specific force acting in a direction to take out the cell from the micropore; and subsequently introducing second cells into the cell selection area to bring these second cells into contact with the first cells remaining in the micropores; switching over to the DC pulse power supply, by the power supply-switchover mechanism; and applying the DC pulse voltage to effect cell fusion between the first cell remaining in the micropore and the second cell contacting thereto.

The cell selection method of the present invention is also a cell selection method, wherein the above-mentioned specific force acting in a direction to take out the cell from the micropore is any one, or a combination of two or more of items of, a gravitational force, a magnetic force, and a fluid delivery force of a solution to be introduced into the cell selection vessel, acting in a direction to take out the cell from the micropore, and a dielectrophoretic force acting in a direction to take out the cell from the micropore, through an application of the second AC voltage from the second AC power supply which has been switched over to by the power supply-switchover mechanism.

Furthermore, the cell selection method of the present invention is a cell selection method, wherein the above-mentioned specific force is a fluid delivery force acting in a direction to take out the cell from the micropore, and cells are selected into cells of which the specific substance on the surface of the cell is not or is weakly bound to the recognition molecule, and cells of which the specific substance on the surface of the cell is strongly bound to the recognition molecule, by changing the strength of the fluid delivery force through variation of the rate of the fluid delivery velocity of a solution to be introduced into the cell selection vessel.

The cell selection method of the present invention is also a cell selection method, wherein the above-mentioned specific force is a dielectrophoretic force acting in a direction to take out the cell from the micropore, and cells are selected into cells of which the specific substance on the surface of the cell is not or is weakly bound to the recognition molecule, and cells of which the specific substance on the surface of the cell is strongly bound to the recognition molecule, by changing the strength of the dielectrophoretic force through variation of the magnitude and/or the frequency of the second AC voltage from the second AC power supply.

The cell selection method of the present invention is also a cell selection method, including capturing cells taken out from the above-mentioned micropores, onto the surface of the electrode on the side of the cell selection area, and/or the surface of the micropored plate-like insulating material disposed on the electrode on the side of the insulating material, where the cationic polymer has been provided.

The cell selection method of the present invention is also a cell selection method, including; immobilizing a recognition molecule which can recognize a specific substance of the above-mentioned first cell, onto a surface of the above-mentioned second cell; and binding the first cell to the second cell through a bond between the specific substance and the recognition molecule.

The cell selection method of the present invention is also a cell selection method, including fusing the first cell and the second cell by applying the DC pulse voltage while applying the specific force acting in a direction to take out the cell from the micropore.

The cell selection method of the present invention is also a cell selection method, wherein the above-mentioned specific substance is an antibody on the surface of the cell, and the above-mentioned recognition molecule is an antigen.

Moreover, another aspect of the cell selection apparatus of the present invention is a cell selection apparatus including oppositely facing first and second electrodes, and a power supply apparatus for applying an AC voltage to these electrodes, wherein an insulating material having micropores is disposed on a surface on the second electrode side of the first electrode, a portion but for an electrode exposure site in contact with these micropores is covered with the insulating material, the first and second electrodes are disposed so that the insulating material and the second electrode are separated, a recognition molecule is disposed on the electrode exposure site, and the power supply apparatus is capable of applying two types of AC voltages having different frequencies to the first and second electrodes.
Hereunder is a more detailed description of the present invention, with reference to the drawings.

The cell selection apparatus of the present invention is a cell selection apparatus including: a cell selection vessel which has a pair of electrodes formed from oppositely disposed conductive members and a sheet-like insulating material disposed between the pair of electrodes via a sheet-like spacer and having a plurality of micropores piercing in the direction of the oppositely disposed electrodes, with the insulating material being disposed on a surface of either one of the electrodes, and a recognition molecule bindable to a specific substance being disposed on a bottom face of the micropore; and a power supply for applying a voltage to the pair of electrodes, wherein the power supply includes a cell-immobilization power supply and a cell-taking power supply, otherwise alternatively, the power supply may include a cell-immobilization power supply, a cell-taking power supply, and a cell-fusion power supply.

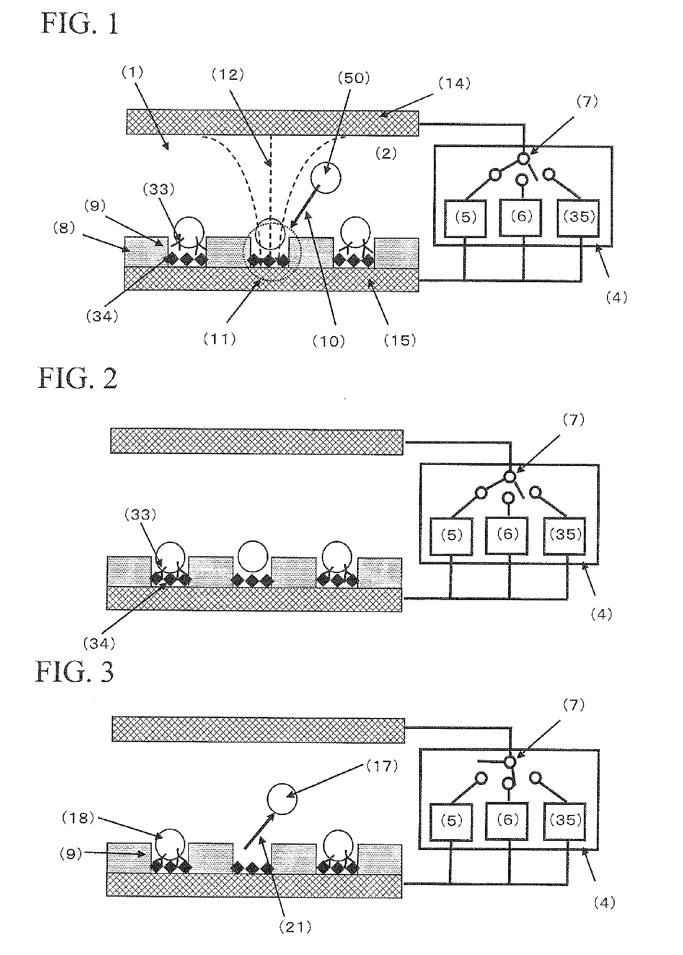

Here, firstly, in order to explain the cell selection apparatus and the cell selection method of the present invention, the outline of a typical cell selection method using the cell selection apparatus of the present invention, that is, a method for selecting cells by introducing a cell solution into a cell selection area, is described with reference to FIG. 1 to FIG. 3.

The procedure of the cell selection method of the present invention is shown in the order of FIG. 1, FIG. 2, and FIG. 3. First, as shown in FIG. 1, a cell solution (2) containing cells (50) is poured into a cell selection area (1), and a power supply-switchover mechanism (7) is connected to a first AC power supply (5) for applying a first AC voltage. The first AC power supply serves as a cell-immobilization power supply. At this time, cells are moved toward micropores (9) formed in an insulating material (8) and immobilized. A force acting to move a cell toward the micropore is referred to as a positive dielectrophoretic force (10) in this invention. As shown in FIG. 1, the positive dielectrophoretic force means a force to move a dielectric particle such as a cell in a direction toward a site (11) where electric force lines (12) are concentrated (that is, a direction toward the micropore) if there is any such a site, like a case where an upper electrode (14) and a lower electrode (15) covered with micropores (9) are provided, when an AC voltage having a specific frequency is applied between these electrodes. Generally speaking, the dielectrophoretic force is proportional to the volume of a dielectric particle, a difference between the dielectric constant of the dielectric particle and the dielectric constant of the solution, and the square of the applied voltage. A positive dielectrophoretic force is generated when an AC voltage having a high frequency (for example, 30 kHz or higher) is applied to cells, or such a substance.

Next, as shown in FIG. 2, cells immobilized in the micropores are brought into contact with the bottom faces of the micropores, thereby causing a binding reaction between a specific substance (33) on a cell surface and a recognition molecule (34) disposed on the bottom face of a micropore (an electrode exposure site where the electrode is in contact with the micropore of the first electrode, in another aspect of the cell selection apparatus of the present invention). Subsequently, as shown in FIG. 3, the first AC power supply (5) is switched over to the second AC power supply (6) by the power supply-switchover mechanism (7), and a second AC voltage is applied. The second AC power supply serves as a cell-taking power supply. At this time, cells inside the micropores are affected by a negative dielectrophoretic force (21). Here, the negative dielectrophoretic force means a force acting in a direction to take out a cell from the micropore (9). Generally speaking, a negative dielectrophoretic force is generated when an AC voltage having a low frequency (for example, lower than 20 kHz) is applied to cells, or such a substance. This makes it possible to forcibly take out cells from the micropores under the negative dielectrophoretic force. At this time, the cell selection can be performed among the entire population of cells immobilized in the micropores, by taking out a cell (17) of which the specific substance on the surface of the cell is not or is weakly bound to the recognition molecule, from the micropore, or by leaving a cell (18) of which the specific substance on the surface of the cell is strongly bound to the recognition molecule, behind in the micropore, under the binding force between the specific substance on the surface of the cell and the recognition molecule. The first AC power supply and the second AC power supply may be either different power supplies, or the same power supply used by switching the voltage or the frequency, as long as they respectively have functions to apply the first AC voltage and the second AC voltage.

That is, an aspect for performing the cell selection in the present invention includes the steps of; introducing cells into the cell selection area; immobilizing these cells in the micropores by applying the first AC voltage; thereafter, effecting a binding reaction between a specific substance (for example, an antibody) on a surface of the cell and a recognition molecule (for example, an antigen); switching the first AC power supply over to the second AC power supply by the power supply-switchover mechanism; and applying a second AC voltage, making it possible, among the entire population of the cells, to take out a cell of which the specific substance on the surface of the cell is not or is weakly bound to the recognition molecule, from the micropore; otherwise alternatively, to leave a cell of which the specific substance on the surface of the cell is strongly bound to the recognition molecule, behind in the micropore; as well as making it possible to select cells of which the specific substance on the surface of the cell is not or is weakly bound to the recognition molecule, and cells of which the specific substance on the surface of the cell is strongly bound to the recognition molecule; and to set the binding force serving as a selection criterion at discretion by changing the magnitude of the voltage from the second AC power supply.

Here, the strength of the binding force referred to in the present invention means a strength in either one or both cases where the binding force of a recognition molecule per se to the specific substance on the cell surface is strong, and where the number of specific substances on the surface of the cell is large, consequently contributing to a strong binding force to the recognition molecule.

Next is a detailed description of the structure of the cell selection apparatus of the present invention, with reference to the drawings.

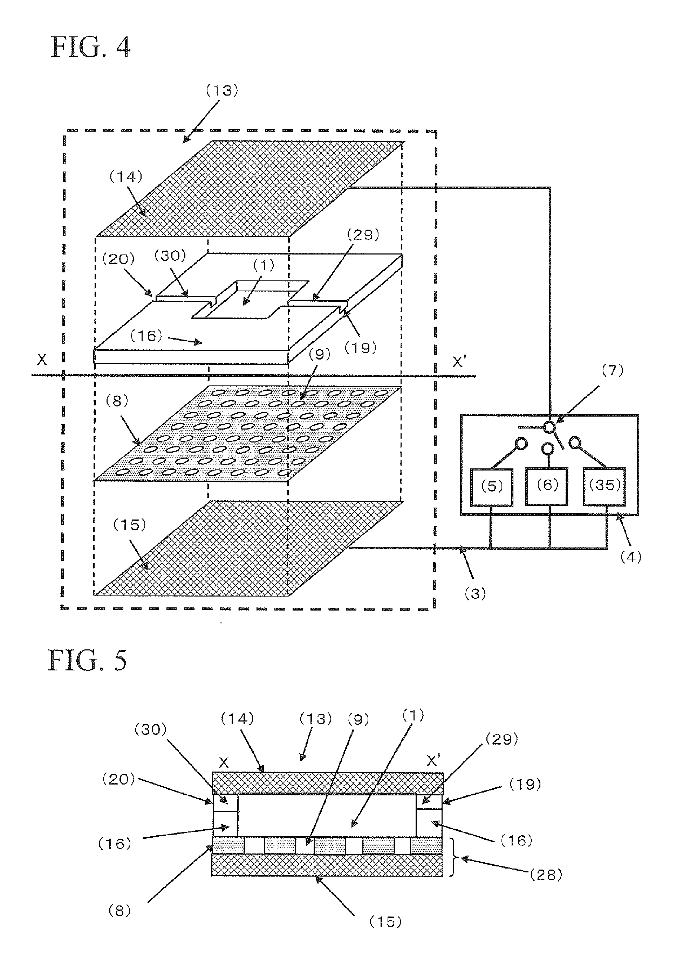

FIG. 4 is a conceptual diagram of the cell selection apparatus of the present invention. The cell selection apparatus of the present invention includes a cell selection vessel (13) and a power supply (4).

As shown in FIG. 4, the cell selection vessel has a structure in which a cell selection area (1) is retained by disposing a spacer (16) between an upper electrode (14) and a lower electrode (15), and an insulating material (8) formed with micropores (9) is disposed on the side of the cell selection area of the lower electrode.
In another aspect of the cell selection apparatus of the present invention, the lower electrode of FIG. 4 on which the insulating material (8) formed with the micropores (9) is disposed corresponds to the first electrode, while the upper electrode disposed apart from this insulating material corresponds to the second electrode. However, in the cell selection apparatus of the present invention which immobilizes or take outs cells through an application two kinds of AC voltages, these electrodes may be disposed upside down, or may also be horizontally disposed rather than the vertical arrangement. The insulating material (8) and the electrode (14) are set apart by disposing the spacer (16) between the insulating material and the electrode, as a result of which the cell selection area (1) to hold a liquid can be retained. However, for example, the spacer can be substituted by having a surface tension by bringing the electrodes closer, or by having a space by pasting the electrodes onto opposing surfaces in a box.

The materials of the upper electrode and the lower electrode are not specifically limited as long as they are electrically conductive and chemically stable members. It is possible to use a metal such as platinum, gold, and copper, an alloy such as a stainless steel, or a glass substrate deposited with a film of a transparent conductor material such as ITO (Indium Tin Oxide), although it is preferable for the observation of the cell selection, to use a glass substrate deposited with a film of a transparent conductor material such as ITO, as an electrode. In addition, a gold electrode is more preferred to immobilize a recognition molecule bindable to a specific substance exclusively onto the micropore portion. In this case, it is possible, by utilizing a gold-thiol bond, or the like, to attach the recognition molecule exclusively to portion where the gold electrode is exposed to the surface. The gold-thiol bond is a firm bond which forms a densely oriented monomolecular film through bonding between a thiol compound having a SH group on its molecular end and a surface of a metal such as gold. For example, if the recognition molecule bindable to the specific substance is an antigen, the binding force of the gold-thiol bond is supposed to be about a million times stronger than the binding force of an antigen-antibody reaction, making it possible to more firmly immobilize the recognition molecule onto the bottom face of the micropore. In the case of the gold-thiol bond, it is possible to cleave the gold-thiol bond by applying an appropriate voltage to the electrodes of the cell selection vessel, so that the cell selection vessel can be readily reused. Furthermore, when utilizing the gold-thiol bond, it is also possible to immobilize gold nanoparticles onto the surface of an electrode such as an ITO transparent electrode or the like, through a chemical bond or physical adsorption, rather than using a gold electrode.

The dimensions of the areas of the upper electrode and the lower electrode are not specifically limited, although preferred is, for example, a size of about length of 70 mm × width of 40 mm × thickness of 1 mm for the sake of easy handling. The upper electrode and the lower electrode of the cell selection vessel are connected with a power supply (4) via a conductive line (3). The power supply (4) includes a first AC power supply (5) for applying a first AC voltage, a second AC power supply (6) for applying a second AC voltage, and a DC pulse power supply (35) for applying a DC pulse voltage, to between the upper electrode and the lower electrode. The first AC power supply, the second AC power supply, and the DC pulse power supply can be used by being appropriately switched over therebetween by the power supply-switchover mechanism (7). The first power supply serves as a cell-immobilization power supply, the second power supply serves as a cell-taking power supply, and the DC pulse power supply serves as a cell-fusion power supply. Here, either one or both of the second power supply and the DC pulse power supply may be set as required. Even if the power supply (4) includes the first AC power supply (5) alone, there is no deviation from the gist of the present invention.

If the power supply includes the first AC power supply alone, the power supply-switchover mechanism (7) also becomes unnecessary, in which case the first AC power supply can be directly connected to the upper electrode and the lower electrode. In addition, the power supply-switchover mechanism is preferably able to pick up any one or two of the first AC power supply, the second AC power supply, and the DC pulse power supply, and be connected to the picked up one. By so doing, for example, by connecting to the DC pulse power supply while keeping the second AC power supply connected, it becomes possible to perform the cell fusion by applying the DC pulse voltage while applying the negative dielectrophoretic force acting in a direction to take out the cell from the micropore, and thereby the cell selection efficiency can be improved.

The spacer is provided to avoid a direct contact between the upper electrode and the lower electrode, and has a through hole which constitutes a cell selection area for retaining a space to pour the cell solution into the cell selection vessel. The material thereof may be any insulating substance, for example, including a glass, ceramics, and a resin. In addition, in order to introduce cells into or discharge cells from the cell selection vessel, the spacer is provided with an inlet channel (29) for introducing cells, an inlet port (19) communicated thereto, an outlet channel (30) for discharging cells, and an outlet port (20) communicated thereto. In this manner, the spacer is for separating the electrode and the insulating material and for holding a liquid. For example, the spacer can be substituted by having a surface tension by bringing the electrodes closer, or by having a space by pasting the electrodes onto opposing surfaces in a box.

In the insulating material (8) are formed micropores (9). The material of the insulating material (8) is not specifically limited as long as it is an insulating substance such as a glass, ceramics, and a resin. However, preferred is a relatively readily-processable material such as a resin because it is necessary to form the piercing micropores therein. The means for forming micropores piercing a resin can be achieved by a known method such as a method in which laser beams are irradiated onto positions of micropores to be formed, and a method in which molding is performed with use of a mold having pins for forming through holes in the positions of micropores. Moreover, when a UV curable resin or the like is used for the insulating material, piercing micropores can be formed by using an exposure photomask printed with a pattern corresponding to the micropores, through typical photolithography (exposure) and etching (development). When forming a plurality of micropores in the insulating material, it is preferable to use a UV curable resin for an insulating material and to form micropores by typical photolithography and etching methods.

The shape and the size of the micropores are not specifically limited. However, in the case of the cell selection apparatus of the present invention, because more effective cell selection can be achieved by immobilizing a single cell per each micropore, it is preferable that the diameter of a maximum circle inscribed in the planar shape of a micropore be within a range of the same to twice the diameter of a cell to be immobilized on the micropore (about one micrometer to tens of micrometers although it varies depending on each cell), and that the depth of the micropore be the same or smaller than the diameter of the cell to be immobilized on the micropore.

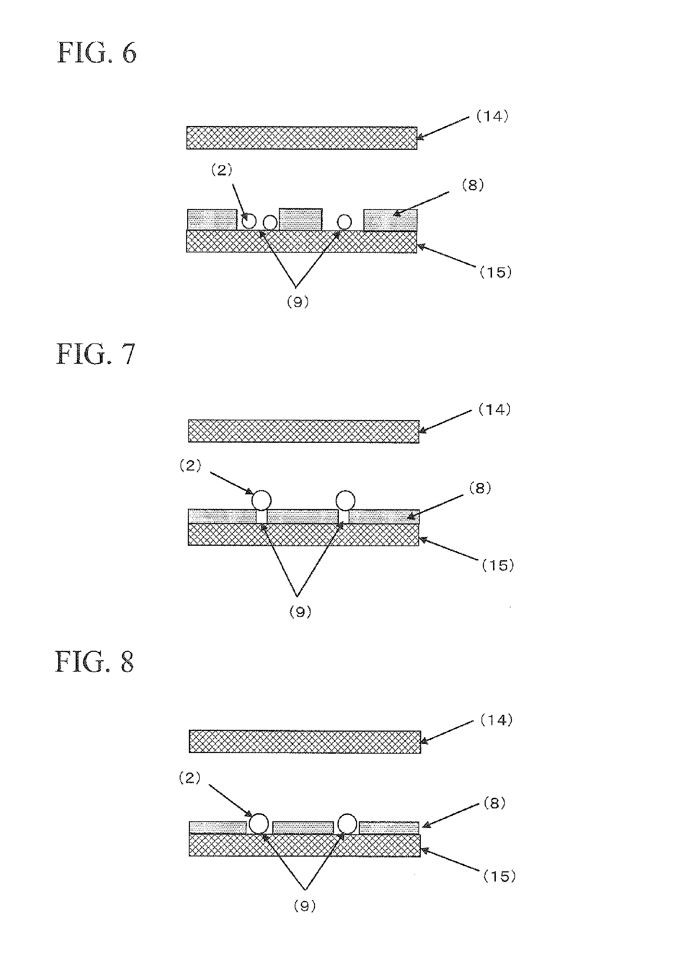

The reason will be explained more in detail with drawings. As shown in FIG. 6, if the diameter of a maximum circle inscribed in the planar shape of a micropore is larger than twice the diameter of a cell to be immobilized on the micropore, a plural number of cells would enter the micropore, disabling the effective cell selection. Similarly, if the depth of the micropore is greater than the diameter of a cell to be immobilized on the micropore, a plural number of cells would enter the micropore, disabling the effective cell selection.

Moreover, as shown in FIG. 7, if the diameter of a maximum circle inscribed in the planar shape of a micropore is smaller than the diameter of a cell to be immobilized on the micropore, the cell immobilized on the micropore would not contact with the bottom face of the micropore, disabling the binding between the recognition molecule disposed on the bottom face of the micropore and the specific substance on the surface of the cell, and consequently disabling fulfillment of the requirement of the present invention.

However, as shown in FIG. 8, if the diameter of a maximum circle inscribed in the planar shape of a micropore is the same to twice greater than the diameter of a cell to be immobilized on the micropore, and if the depth of the micropore is the same or smaller than the diameter of the cell to be immobilized on the micropore, it becomes more likely that approximately one cell can enter one micropore, and the cell immobilized on the micropore can reliably contact with the bottom face of the micropore, enabling the binding between the recognition molecule disposed on the bottom face of the micropore and the specific substance on the surface of the cell, and consequently enabling fulfillment of the requirement of the present invention, so that more effective cell selection can be achieved.

The cell selection apparatus of the present invention is capable of more effective cell selection if a single cell can be immobilized per each micropore. Therefore, it is preferable that a plurality of micropores are formed in the insulating material so that any adjacent micropores are positioned at equal intervals in the surface of the insulating material, that is, as shown by the micropores (9) formed in the insulating material (8) of FIG. 4, it is preferable that a plurality of micropores are formed in an array like arrangement in the surface of the insulating material. Here, the term array like arrangement means an arrangement where lengthwise and widthwise intervals between micropores are approximately the same.

By disposing the micropores in an array like arrangement, approximately equal levels of electric fields are generated in all micropores by a voltage applied between the electrodes. Therefore, the respective micropores become equally likely to immobilize cells, making it more likely that a single cell be immobilized per each micropore. In addition, for immobilizing a single cell per each micropore, it may be sometimes inappropriate if the interval between micropores formed in an array like arrangement is too narrow or too wide. If the interval between micropores is too narrow, each micropore is more likely to immobilize a plural number of cells, as a result of which the number of micropores without a cell would increase sometimes. Moreover, if the interval between micropores is too wide, cells are left behind between micropores, as a result of which the number of micropores without a cell would increase sometimes. Accordingly, more specifically, it is preferable that the interval between adjacent micropores be within a range of half to six times the diameter of a cell to be immobilized on each micropore, and furthermore it is more preferable that the interval between micropores be about the same to twice the diameter of a cell to be immobilized.

Furthermore, the shape of the micropore of the present invention is not limited to a circular shape, and may be a polygonal shape such as a triangular or quadrangular shape. In the case of a polygonal shape such as a triangular or quadrangular shape, the electric lines of force are more concentrated at the corners, providing a merit in that the dielectrophoretic force becomes stronger than that of circular micropores so that cells are more likely to be immobilized on micropores. However, if micropores are disposed in an array like arrangement, it would be more likely that a single cell be immobilized per each micropore by equally effecting dielectrophoretic forces from front, back, left, and right micropores. Therefore, the shape of a micropore is preferably point symmetric, and more preferably square.

FIG. 5 is a schematic diagram showing a cross-sectional view taken along the line XX' of the cell selection vessel of FIG. 4. The means for pasting the upper electrode (14), the spacer (16), the insulating material (8), and the lower electrode (15) as in FIG. 5 can be achieved by a known method, such as a method of gluing them with an adhesive, a method of heat-sealing them under a pressurized state, and a method of pasting them by pressure using a spacer made from a resin such as surface-cohesive PDMS (poly-dimethylsiloxane) or a silicone sheet. By so doing, the cell selection area (1) shown in FIG. 5 can be formed.

In the cell selection vessel of the present invention, a recognition molecule bindable to the specific substance is disposed on the bottom face of the micropore. Here, the combination of the specific substance and the recognition molecule is not specifically limited as long as the specific substance and the recognition molecule can be selectively bound with each other. For example, a receptor is preferred as a recognition molecule for the purpose of selecting a cell of which a ligand is presented as a specific substance on the surface of the cell, a lectin is preferred as a recognition molecule for the purpose of selecting a cell of which a sugar chain is presented as a specific substance on the surface of the cell, an antigen is preferred as a recognition molecule for the purpose of selecting a cell of which an antibody is presented as a specific substance on the surface of the cell, and an antibody is preferred as a recognition molecule for the purpose of selecting a cell of which an antigen is presented as a specific substance on the surface of the cell. Moreover, the method of disposing the recognition molecule on the bottom face of the micropore is not specifically limited as long as the recognition molecule can be immobilized at least on the bottom face of the micropore. It is possible to utilize a chemical bond such as an amide bond, a biotin-avidin bond, or a thiol bond, or it is also possible to effect a physical adsorption by introducing a solution which contains a recognition molecule bindable to the specific substance or a recognition molecule bound to a protein, or the like, into the cell selection vessel.

A molecule, a protein, or such a substance for facilitating the immobilization of the recognition molecule to the bottom face of the micropore may be bound to a site of the recognition molecule other than the site at which the specific substance is bound to the recognition molecule. The molecule for facilitating the immobilization of the recognition molecule to the bottom face of the micropore can be exemplified by biotin, avidin, an amino group, a thiol group, or a disulfide group, as well as an alkyl chain or a polyethylene glycol chain which contains these groups. Moreover, the protein for facilitating the immobilization of the recognition molecule to the bottom face of the micropore can be exemplified by BSA (Bovine Serum Albumin), BCP (Blue Carrier Immunogenic Protein), and KLH (Keyhole Limpet Hemocyanin).

In addition, it is also possible, after arranging the recognition molecule in this manner, to bring a molecule or a protein which would not involve the binding reaction between the specific substance on the surface of the cell and the recognition molecule, into contact with the bottom face of the micropore or the surface of the insulating material, to effect a chemical bond or physical adsorption therebetween.

Here, the action to bring a molecule or a protein which would not involve the binding reaction between the specific substance on the surface of the cell and the recognition molecule, into contact with the surface of the insulating material, to effect a chemical bond or physical adsorption, is referred to as blocking. The blocking is performed for the purpose of preventing nonspecific adsorptions of cells and proteins to the surface, by covering the surface with a molecule or a protein which would not involve the binding reaction between the specific substance on the surface of the cell and the recognition molecule.

A typical molecule for use in the blocking can be exemplified by an alkyl chain, a polyethylene glycol chain, or such a substance, as well as a molecule containing them. A protein solution for use in the blocking can be exemplified by skim milk, BSA, and casein.

Such a protein is nonspecifically adsorbed onto a surface of a plastic tube, a microplate, a glass bead, an insulating material, or the like, due to surface charges or hydrophobic interactions. In addition, for adsorbing a protein, it is generally supposed to be preferable that the pH of the solvent be more alkaline than the isoelectric point of the protein. Therefore, phosphate buffered saline (PBS), carbonate buffer (Na₂CO₃ or NaHCO₃), or the like is used as a solvent.

Since the specific substance presented on the surface of the cell and the recognition molecule can be more selectively bound with each other by such a blocking operation, this cell selection method is able to improve the selection efficiency.

In addition, the power supply in the present invention includes a first AC power supply, and may also includes a second AC power supply and a DC pulse power supply, as required.

The first AC power supply is a cell-immobilization power supply, which is used for moving cells toward the micropores formed in the insulating material and for immobilizing them. Moreover, the second AC power supply is a cell-taking power supply, which is used for selecting cells by taking them out from the micropores formed in the insulating material under a dielectrophoretic force. Furthermore, the DC pulse power supply is a cell-fusion power supply, which is used for performing an electrical cell fusion between a remaining first cell which has been left behind in the micropore through the selection of the first cells by the present invention and a second cell which has been subsequently introduced.

The first AC power supply for use in the cell selection apparatus of the present invention is not specifically limited as long as cells can be immobilized in the micropores. For example, it is possible to employ an AC power supply which can output an AC voltage having a sine wave, a rectangular wave, a triangular wave, a trapezoid wave, or such a waveform, with a peak voltage of about 1 V to 20 V and a frequency of about 30 kHz to 3 MHz, and preferably about 1 MHz to 3 MHz.

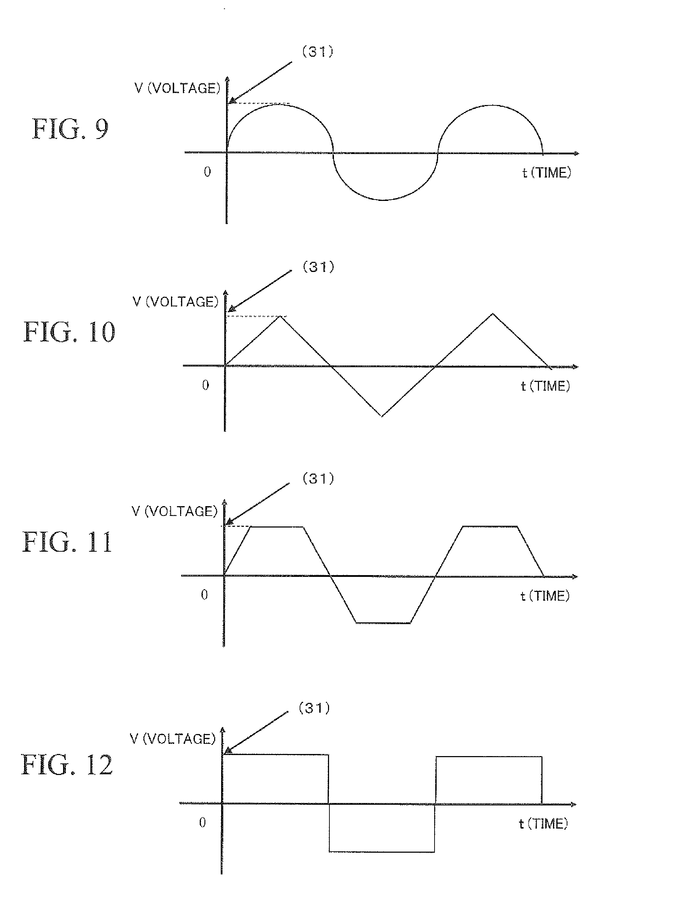

When using the cell selection apparatus of the present invention, more effective cell selection can be achieved by immobilizing a single cell per each micropore, and it is preferable that the waveform of the AC voltage for immobilizing a single cell per each micropore is rectangular. The reason is that, as shown in FIG. 9 to FIG. 12, the AC voltage can more instantaneously reach the set peak voltage (31) when the waveform is rectangular (FIG. 12) as compared to cases of the sine wave (FIG. 9), the triangular wave (FIG. 10), and the trapezoid wave (FIG. 11). Therefore, cells can be promptly moved, as a result of which cells are less likely to overlappingly enter one micropore, and accordingly it is more likely that a single cell be immobilized per each micropore. In addition, a cell can be regarded as a capacitor from an electrical point of view, because of which a current can hardly flow through a cell inside a micropore while the peak voltage of a rectangular waveform is unchanged. As a result, electric lines of force would be hardly generated, and thus a dielectrophoretic force would be hardly generated in a micropore containing a cell. For this reason, once a cell has entered a micropore, the other cells are less likely to enter the micropore, as the other cells will serially enter vacant micropores where electric lines of force are generated and dielectrophoretic forces are generated.

The waveform of the AC voltage for use in the cell selection apparatus of the present invention preferably has no DC component. This is because, due to an electrostatic force generated by a DC component, cells are moved by receiving a unidirectionally biased force, which makes it difficult to immobilize the cells in the micropores under a dielectrophoretic force. This is also because, ions contained in a cell-suspended solution cause an electric reaction on the surface of an electrode to thereby produce heat, as a result of which cells exhibit a thermal motion, and therefore the cell movement eventually becomes uncontrollable under the dielectrophoretic force, making it difficult to draw the cells to the micropores.

The second AC power supply for use in the cell selection apparatus of the present invention is not specifically limited as long as cells can be taken out from the micropores. For example, it is possible to employ an AC power supply which can output an AC voltage having a sine wave, a rectangular wave, a triangular wave, a trapezoid wave, or such a waveform, with a peak voltage of about 1 V to 20 V and a frequency of less than 20 kHz, and preferably about 5 kHz to 10 kHz.

The DC power supply for use in the cell selection apparatus of the present invention is not specifically limited as long as it can generate a DC pulse voltage capable of effecting a cell fusion between a mutually contacted first cell and second cell within or in the vicinity of a micropore. There is no specific limitation as long as the DC pulse power supply can output a DC pulse voltage of about 50 V to 1000 V with a pulse width of about 10 µsec to 50 µsec, for example.

In addition, in the cell selection apparatus of the present invention, a cationic polymer may also be provided on a surface of the electrode which faces the cell selection area, and/or a surface of the micropored plate-like insulating material disposed on the electrode on the side of the insulating material, of the cell selection vessel. The cationic polymer brings about an ionic bond between a negative charge on the surface of the electrode or the insulating material and a negative charge of a sialic acid residing on the surface of the cell, with the aid of a positive charge of the cationic polymer. By having this aspect, cells taken out from the micropores by the cell selection method of the present invention can be adhered to the surface of the electrode which faces the cell selection area, and/or the surface of the micropored plate-like insulating material disposed on the electrode on the side of the insulating material. As a result, the cells taken out from the micropores are less likely to be re-immobilized in the micropores, and thereby the selection efficiency can be improved.

The cationic polymer can be exemplified by a polylysine, a polylysine derivative, an amino group-containing polymer, or the like. There is no specific limitation as long as the cationic polymer is capable of adhering cells, although it is preferable to have less toxicity to cells and higher adhesiveness to cells

The cationic polymer can be taken out by applying an appropriate voltage to the electrodes of the cell selection vessel so that the cell selection vessel can be readily reused.

Next is a description of the cell selection method in the present invention.

The cell selection method of the present invention is a cell selection method using the above-mentioned cell selection apparatus, including; introducing cells into the cell selection area; immobilizing these cells in the micropores under a dielectrophoretic force by applying the first AC voltage from the first AC power supply; effecting a binding reaction between the specific substance (for example, an antibody) on the surface of the cell and the recognition molecule (for example, an antigen) disposed on the bottom face of the micropore; and thereafter, taking out a cell of which the specific substance on the surface of the cell is not or is weakly bound to the recognition molecule, among the entire population of the cells, from the micropore, under a specific force acting in a direction to take out the cell from the micropore; otherwise alternatively, leaving a cell of which the specific substance on the surface of the cell is strongly bound to the recognition molecule, among the entire population of the cells, behind in the micropore, under a specific force acting in a direction to take out the cell from the micropore.

Here, the specific force means a force acting in a direction to take out the cell from the micropore. There is no specific limitation as long as the specific force is approximately equivalent to the binding force (approximately pN to nN level) between the specific substance on the surface of the cell and the recognition molecule on the bottom face of the micropore. The specific force may be a fluid delivery force of a solution to be introduced into the cell selection vessel, a dielectrophoretic force acting in a direction to take out the cell from the micropore, through an application of the second AC voltage from the second AC power supply which has been switched over to by the power supply-switchover mechanism, a gravitational force acting in accordance with the mass weight of the cell immobilized in the micropore, or a magnetic force if a magnetic fine particle or such a material is adhered on the surface of the cell.

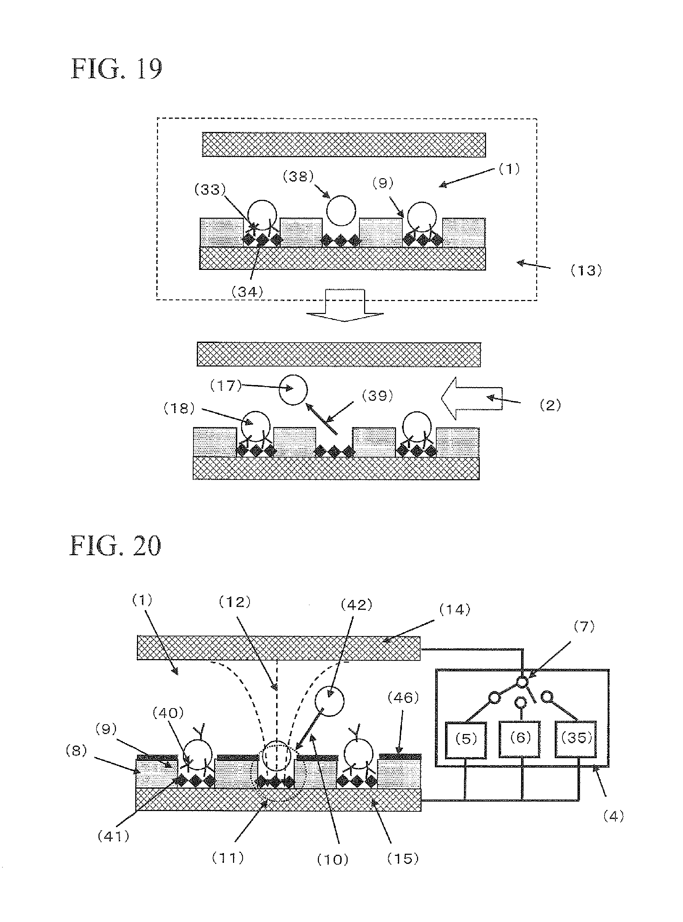

As shown in FIG. 19, in a case where a fluid delivery force (39) is effected on a cell in a direction to take out the cell from the micropore as a specific force acting in an opposite direction against the binding force between the specific substance (33) on the surface of the cell and the recognition molecule (34) on the bottom face of the micropore, a cell solution (2) which does not contain any cell can be introduced into the cell selection area (1).

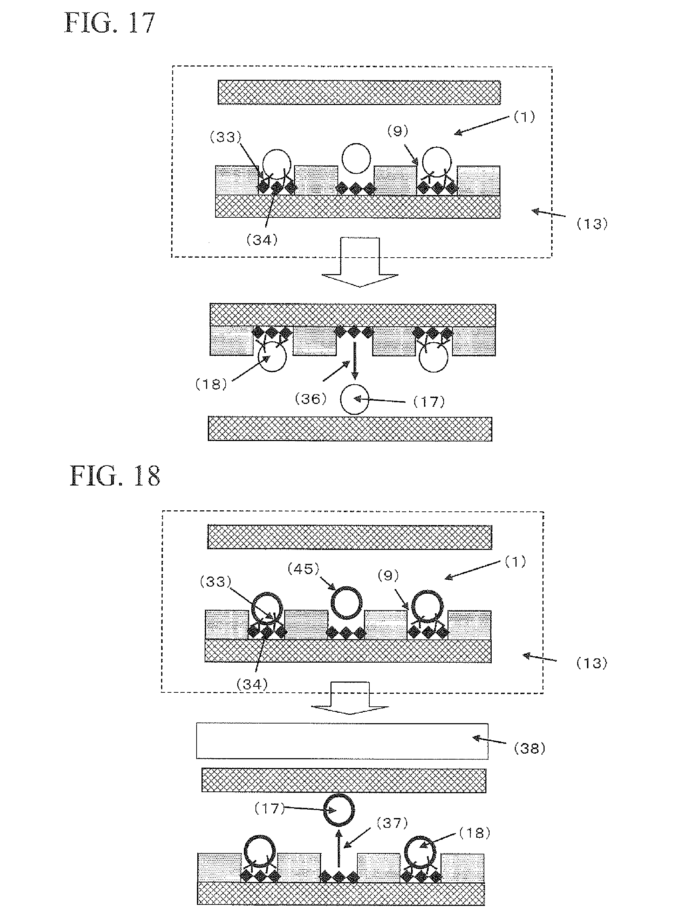

Moreover, FIG. 17 shows a case where a gravitational force (36) is effected on a cell in a direction to take out the cell from the micropore as a specific force acting in an opposite direction against the binding force between the specific substance (33) on the surface of the cell and the recognition molecule (34) on the bottom face of the micropore. As shown in FIG. 17, for immobilizing cells in the micropores (9) of the cell selection area (1), the electrode disposed with the micropored plate-like insulating material can be set on the lower side, and after binding the specific substance (33) on the surface of the cell and the recognition molecule (34) of the bottom face of the micropore, the cell selection vessel (13) can be inverted so that the electrode disposed with the micropored plate-like insulating material be set on the upper side.

In addition, FIG. 18 shows a case where a magnetic force (37) is effected on a cell in a direction to take out the cell from the micropore as a specific force acting in an opposite direction against the binding force between the specific substance (33) on the surface of the cell and the recognition molecule (34) on the bottom face of the micropore. As shown in FIG. 18, using a cell having magnetic fine particles (45) adhered on the surface, the electrode disposed with the micropored plate-like insulating material can be set on the lower side of the cell selection vessel (13), and a magnetic body (38) can be set on the upper side of the cell selection vessel. The method for adhering magnetic fine particles onto a cell may be a known method utilizing physical adsorption or a chemical bond such as a biotin-avidin bond.

Among these specific forces, regarding the fluid delivery force acting in a direction to take out the cell from the micropore, the strength of the fluid delivery force can be readily changed through variation of the rate of the fluid delivery velocity of a solution to be introduced into the cell selection vessel. Moreover, regarding the dielectrophoretic force acting in a direction to take out the cell from the micropore, the strength of the dielectrophoretic force can be readily changed through variation of the magnitude and/or the frequency of the second AC voltage from the second AC power supply. That is, it becomes possible to set at discretion the binding force serving as a selection criterion for selecting cells into cells of which the specific substance on the surface of the cell is not or is weakly bound to the recognition molecule, and cells of which the specific substance on the surface of the cell is strongly bound to the recognition molecule. Accordingly, if a fluid delivery force or a dielectrophoretic force is used as the specific force, it becomes possible for the first time to easily select cells based on the strength of the binding force between a specific substance on the surface of the cell and a recognition molecule, which has been so far difficult with current techniques.

Here, the time for applying the gravitational force, the magnetic force, the fluid delivery force, or the dielectrophoretic force as the specific force is not specifically limited as long as the cell of interest can be selected. Generally, the proportion to cleave the binding force between the specific substance and the recognition molecule increases until a certain period of time when the gravitational force, the magnetic force, the fluid delivery force, or the dielectrophoretic force is applied, and is eventually saturated. Therefore, only within this certain period of time, it is possible to set at discretion the selection criterion for selecting cells of which the specific substance on the surface of the cell is strongly bound to the recognition molecule, according to the time for applying the gravitational force, the magnetic force, the fluid delivery force, or the dielectrophoretic force.

In the cell selection method of the present invention, the specific force may be any one, or a combination of two or more of items of, the gravitational force, the fluid delivery force, the dielectrophoretic force, and the magnetic force, depending on the kind of cells to be selected, or the binding force between the specific substance on the surface of the cell and the recognition molecule.

In addition, since the cell selection method of the present invention is to take out a cell of which the specific substance on the surface of the cell is not or is weakly bound to the recognition molecule, from the micropore, it is ideal that the diameter of the micropore is larger than the diameter of the cell to be selected. However, from the examination results done by the inventors of the present invention, it was found that the refusion probability increases when the cell fusion is performed by using the cell selection method of this invention. Therefore, it is preferable that the diameter of the micropore and the diameter of the cell be approximately the same. Accordingly, when cell fusion is performed subsequently from the cell selection as will be described later, if the diameter of the micropore and the diameter of the cell are approximately the same, the cell would be nonspecifically adsorbed onto a wall of the micropore, which may make it difficult to take out the cell of which the specific substance on the surface of the cell is not or is weakly bound to the recognition molecule, from the micropore, at the time of the cell selection. In such a case, it is possible to more effectively take out the cell from the micropore by changing the osmotic pressure and/or the specific gravity of the cell-containing solution.

For example, the osmotic pressure of a solvent can be increased by replacing the solvent from a 300 mM mannitol solvent into a 500 mM mannitol solvent, decreasing the diameter of a cell, and thus making it easy to take out the cell from the micropore. Moreover, if the solvent is replaced from mannitol into sucrose, a buoyant force acting on the cell can be generated because the specific gravity of sucrose is greater than the specific gravity of mannitol, thus enhancing the effect to take out the cell from the micropore when using the negative dielectrophoretic force, the fluid delivery force, or the magnetic force. This method is capable of generating a buoyant force on a cell in the same manner also by adding a non-sucrose watersoluble polymer such as ficoll into a solvent.

In the cell selection method of the present invention, such solvent replacement can be performed at any timing. It is possible to replace a solvent with another solvent at the time of cell selection, and after taking out the cell of which the specific substance on the surface of the cell is not or is weakly bound to the recognition molecule, from the micropore, to replace the solvent again back to the original solvent. It is possible to choose at discretion the optimum solvent condition for the cell selection and the optimum solvent condition for the cell fusion.

Hereunder is a more detailed description with an example where cells can be selected by the strength of the binding force between the specific substance of the surface of the cell and the recognition molecule, based on the magnitude of the second AC voltage, that is, the strength of the dielectrophoretic force acting on the cells, with reference to the drawings.
FIGS. 1 to 3 and FIG. 13 show conceptual diagrams of the cell selection method of the present invention.

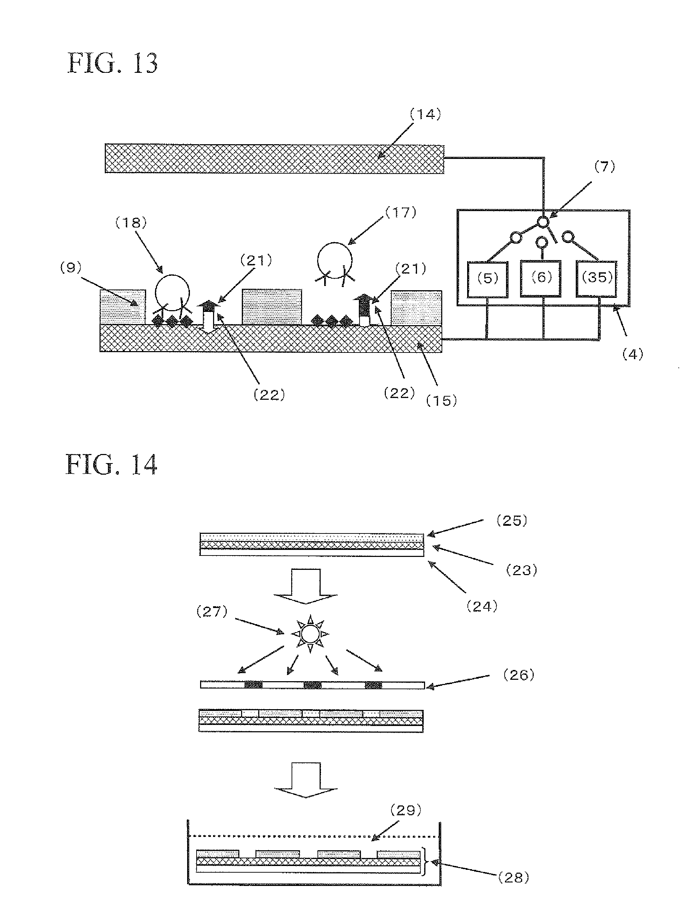

In FIG. 1, cells are introduced into the cell selection area where the recognition molecules (34) bindable to a specific substance are immobilized on the bottom faces of micropores, and these cells are immobilized in the micropores by applying the first AC voltage. Next, as shown in FIG. 2, these cells are brought into contact with the bottom faces of the micropores, to thereby cause a binding reaction between the specific substance (33) on the surface of the cell and the recognition molecule. Subsequently, as shown in FIG. 3, a force to take out a cell from the micropore (negative dielectrophoretic force) is effected by applying a second AC voltage. Here, a detailed description is given with reference to FIG. 13. Of these cells, a cell (18) of which the specific substance on the surface of the cell is strongly bound to the recognition molecule, is left behind in the micropore because the binding force (22) between the specific substance on the surface of the cell and the recognition molecule is stronger than the negative dielectrophoretic force (21). On the other hand, a cell (17) of which the specific substance on the surface of the cell is not or is weakly bound to the recognition molecule, is taken out from the micropores because the negative dielectrophoretic force (21) is stronger than the binding force (22) between the specific substance on the surface of the cell and the recognition molecule. That is, cells can be selected by adjusting the strength of the negative dielectrophoretic force to thereby adjust the selection criterion of the binding force between the specific substance on the surface of the cell and the recognition molecule. Here, the strength of the negative dielectrophoretic force can be controlled by the voltage or the frequency of the second AC power supply, although it is simplest and preferable to control it by the voltage of the second AC power supply. For example, when the voltage of the second AC power supply is within a range of 2.5 V to 10 V, the selection can be achieved with a voltage application time of about 30 to 40 seconds. However, for the purpose of eliminating damage to the cells and suppressing deactivation of the cells, the voltage is preferably as low as possible within an effective range of the dielectrophoretic force.

The above-mentioned procedure enables to choose the strength of the binding force between the specific substance on the surface of the cell and the recognition molecule, based on the magnitude of the second AC voltage, that is, the strength of the dielectrophoretic force.

In addition, the cell selection method of the present invention may also be a cell selection method using the above-mentioned cell selection apparatus, being a cell selection method as mentioned below. That is, the method may include the following steps. First cells are introduced into the cell selection area, and these first cells are immobilized in the micropores by applying the first AC voltage from the first AC power supply. A binding reaction is effected between the specific substance on a surface of the first cell and the recognition molecule. Then, among the entire population of the first cells, a cell of which the specific substance on the surface of the cell is not or is weakly bound to the recognition molecule is taken out from the micropore, under the above-mentioned specific force. Subsequently, second cells are introduced into the cell selection area, and these second cells are brought into contact with the first cells remaining in the micropores. The power supply is switched over to the DC pulse power supply by the power supply-switchover mechanism, and the DC pulse voltage is applied to effect cell fusion between the first cell remaining in the micropore and the second cell contacting thereto.

This aspect enables to conduct cell selection and cell fusion in a successive manner within the same vessel, therefore making it possible to pick up, among the entire population of the first cells (for example, mouse spleen cells), a first cell of which a specific substance (for example, an antibody) on the surface of the cell is strongly bound to a recognition molecule (for example, an antigen), and to perform cell fusion between the first cell and a second cell (for example, mouse cancer cell). For this reason, the number of fused cells greatly decreases, and the proportion of fused cells capable of producing antibodies that are strongly bindable to the antigen increases. Therefore, the cell selection work to pick up fused cells capable of producing antibodies that are strongly bindable to the antigen, after performing the cell fusion, can be remarkably reduced. Moreover, if the number of micropores to immobilize cells is increased (for example, to about one million to 30 million) in the cell selection apparatus of the present invention, a mass of sample cells can be selected and fused in a quite short period of time, based on the evaluation of the binding force between the specific substance on the surface of the cell and the recognition molecule.

That is, there is no need of separately preparing an apparatus and a vessel for selecting cells and an apparatus and a vessel for fusing cells, making it possible to select and fuse a mass of cells more easily in a shorter period of time, as compared to the above-mentioned conventional techniques such as the ELISA method or the method described in Patent Document 1 in which the performance of antibodies produced by each fused cell has to be evaluated after the cell fusion, and methods such as the magnetic bead method, the flow cytometry method, or the methods described in Patent Document 2 to 4 in which the cell fusion has to be performed in a different apparatus and a different vessel after the cell selection.

In addition, the cell selection and the cell fusion can be conducted in a successive manner within the same vessel, making it possible to perform a quick and efficient operation as well as to maintain the activity of the cells. Moreover, it also becomes possible to remarkably reduce the loss of cells occurring during the transfer of cells from the vessel for selecting cells into the vessel for fusing cells. Moreover, there is no need of an expensive and large-scaled apparatus such as with flow cytometry, and the cell selection and the cell fusion can be conducted in a successive manner in an inexpensive and small-scaled apparatus.

Moreover, in the cell selection method of the present invention, first cells remaining in the micropores after the cell selection are forcibly contacted with second cells which have been introduced afterward, by the first AC voltage from the first AC power supply, and the first cells and the second cells are fused as a pair. For this reason, it becomes possible to achieve an extremely high refusion probability (the refusion probability in the present invention examined by the inventors of the present invention was about 20/10000 to 100/10000 without conducting the cell selection). Therefore, even if the number of the first cells remarkably decreases due to the cell selection, it is substantially possible to generate fused cells. In the method described in Patent Document 3 and the method described in Patent Document 4 which are conventional methods, the cell fusion method to be conducted after the cell selection is the PEG method or the electrofusion method which are conventional cell fusion methods, because of which the refusion probability thereof is very low at about 0.2/10000 in general, involving a problem in that it is substantially difficult to generate fused cells. According to the cell selection method of the present invention, it becomes possible for the first time to solve these problems and substantially possible to generate fused cells after the cell selection. Since the works for selecting cells and fusing cells are conducted within the same vessel, cells are less prone to be lost during the replacement of vessels, unlike the conventional manner.

With the cells immobilized on the micropores after the cell selection or the cell fusion in the cell selection apparatus of the present invention, it is possible to readily cleave the bond between the recognition molecule and the specific substance on the surface of the cell through adjustment of the pH or the like of the solution. Unlike the magnetic bead method or the method described in Patent Document 2, there is no need of any complicated process for separating target cells from the magnetic beads or the support, and it becomes possible to readily recover the selected cells or the fused cells.

Furthermore, when a fluid delivery force or a dielectrophoretic force acting in a direction to take out a cell from a micropore is used as the specific force acting in a direction to take out the cell from the micropore in the cell selection method of the present invention, the strength of the fluid delivery force or the dielectrophoretic force can be readily changed through variation of the rate of the fluid delivery velocity of a solution to be introduced into the cell selection vessel, or the magnitude and/or the frequency of the second AC voltage from the second AC power supply. For this reason, it becomes possible to set at discretion the binding force serving as a selection criterion for selecting cells into cells of which the specific substance on the surface of the cell is not or is weakly bound to the recognition molecule, and cells of which the specific substance on the surface of the cell is strongly bound to the recognition molecule. Accordingly, it becomes possible for the first time to easily select cells based on the strength of the binding force between the specific substance on the surface of the cell and the recognition molecule, such as the binding in an antigen-antibody reaction, which has been so far an issue of the above-mentioned current techniques, and to fuse the selected cell with another cell.

In addition, as described in the cell selection apparatus of the present invention, the cell selection method of the present invention may also include capturing cells taken out from the micropores, onto the surface of the electrode on the side of the cell selection area, and/or the surface of the micropored plate-like insulating material disposed on the electrode on the side of the insulating material, where the cationic polymer has been provided. As mentioned above, a cationic polymer has a function to bring about an ionic bond between a negative charge on the surface of the electrode or the insulating material and a negative charge of a sialic acid residing on the surface of the cell, with the aid of a positive charge of the cationic polymer. By having this aspect, cells taken out from the micropores by selection using the cell selection method of the present invention can be adhered to the surface of the electrode which faces the cell selection area, and/or the surface of the micropored plate-like insulating material disposed on the electrode on the side of the insulating material. As a result, the cells taken out from the micropores are less likely to be re-immobilized on the micropores, and it becomes possible to clearly distinguish and select these cells from other cells immobilized on the micropores, thereby improving the selection efficiency.

Moreover, in a case where the cell selection apparatus of the present invention is in an aspect where no cationic polymer is provided on a surface of the electrode which faces the cell selection area, and/or a surface of the micropored plate-like insulating material disposed on the electrode on the side of the insulating material, in the cell selection vessel, the structure may be such that, after cells have been selected into cells taken out from the micropores and cells left behind in the micropores by selection using the cell selection method of the present invention, a solution containing the cationic polymer is introduced into the cell selection area so that cells taken out from the micropores can be adhered to the surface of the electrode which faces the cell selection area, and/or the surface of the micropored plate-like insulating material disposed on the electrode on the side of the insulating material. This aspect makes it possible to minimize the toxicity to cells and to maintain the cell activity, by introducing a solution not containing the cationic polymer into the cell selection area so as to replace the solution inside the cell selection to effect a removal of excessive cationic polymers, after the cells taken out from the micropores have been adhered to the surface of the electrode which faces the cell selection area, and/or the surface of the micropored plate-like insulating material disposed on the electrode on the side of the insulating material, if the toxicity of the cationic polymer to the cells is of concern.

The abovementioned method of capturing cells taken out from the micropores, onto the surface of the electrode on the side of the cell selection area, and/or the surface of the micropored plate-like insulating material disposed on the electrode on the side of the insulating material, where the cationic polymer has been provided, is particularly effective for conducting the cell selection and the cell fusion in a successive manner in the present invention. That is, first cells taken out from the micropores can be prevented from re-entering the micropores, by feeding a solution containing second cells into the cell selection area when introducing the second cells to be fused with the first cells, after cells on the surfaces of which the specific substance is not or is weakly bound to the recognition molecule, among the entire population of the first cells, have been taken out from the micropore, under the specific force. In addition, it becomes possible to selectively fuse the first cells remaining in the micropores, that is, first cells on the surfaces of which the specific substance is strongly bound to the recognition molecule, with the second cells, and thereby the efficiency to select the fused cells after the cell fusion can be further improved.

Hereunder is a more detailed description of an example where fused cells capable of producing antibodies that are more strongly bindable to an antigen are selected, by: selecting mouse spleen cells on the surfaces of which antibodies more strongly bound to the antigen are presented, under the strength of the binding force between the antibody serving as the specific substance on the surface of the mouse spleen cell serving as the first cell and the antigen serving as the recognition molecule, based on the magnitude of the second AC voltage, that is, the strength of the dielectrophoretic force acting in a direction to take out a cell from a micropore; and, in a successive manner after the cell selection, conducting the cell fusion by introducing mouse cancer cells serving as the second cells, with reference to drawings.
FIG. 20 to FIG. 26 show conceptual diagrams of the method of the present invention where the cell selection and the cell fusion are conducted in a successive manner.

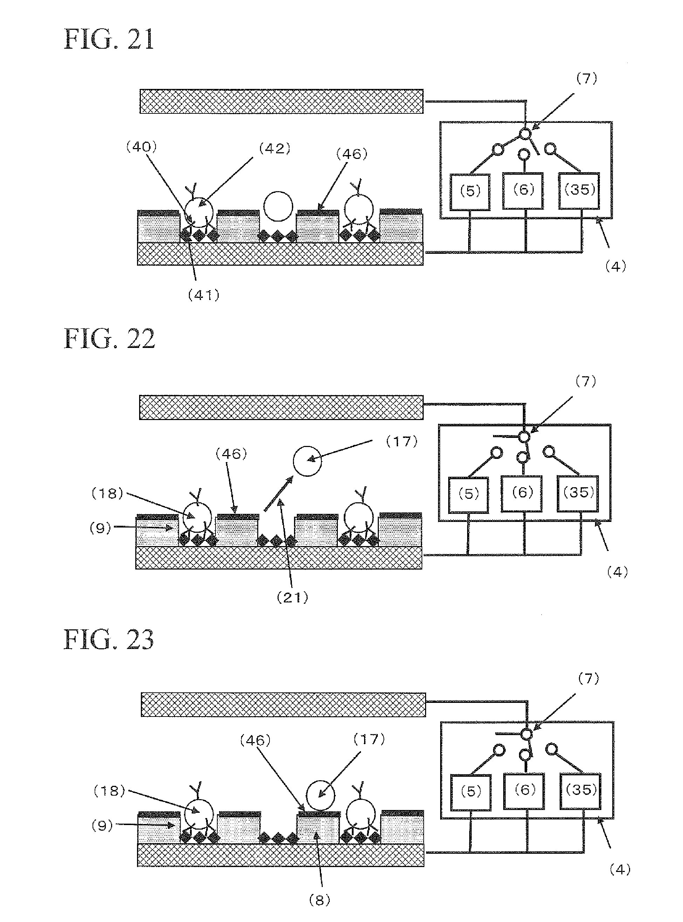

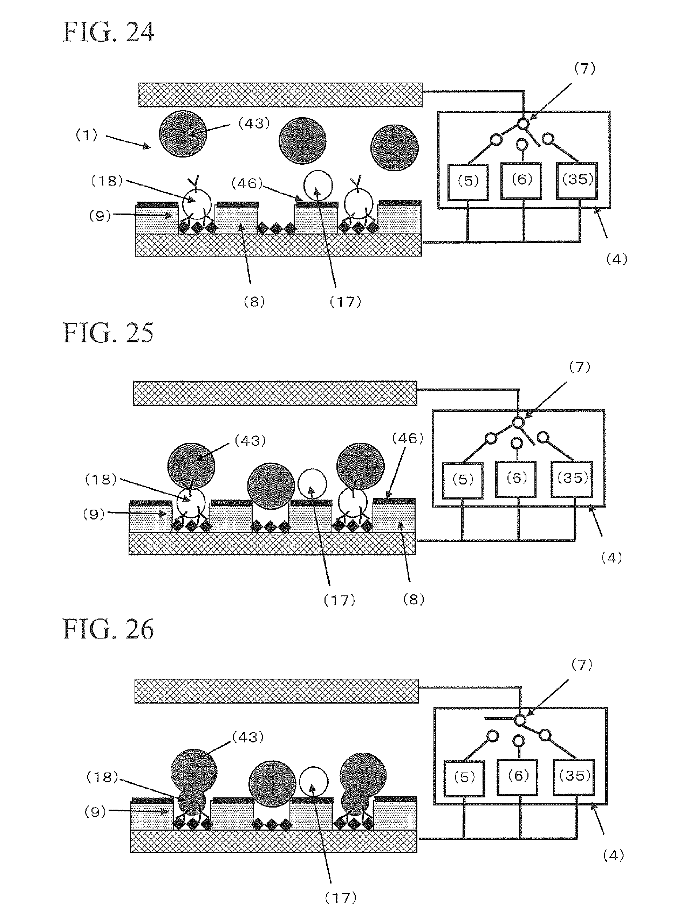

In FIG. 20, mouse spleen cells (42) are introduced into a cell selection area (1) where antigens (41) bindable to a specific antibody (40) are immobilized on the bottom faces of the micropores, and these mouse spleen cells are immobilized in the micropores (9) by applying a first AC voltage from the first AC power supply (5). Next, as shown in FIG. 21, the mouse spleen cells (42) are brought into contact with the bottom faces of the micropores to thereby cause an antigen-antibody reaction between the antibody (40) presented on the surface of the cell and the antigen (41) immobilized on the bottom face of the micropore, thus making a bond therebetween. Subsequently, as shown in FIG. 22, a negative dielectrophoretic force (21) is effected as the force to take out the mouse spleen cells from the micropores, by applying a second AC voltage from the second AC power supply (6). Here, a cell (18) on the surface of which the antibody is strongly bound to the antigen, among the entire population of the mouse spleen cells immobilized in the micropores, is left behind in the micropore because the binding force between the antigen and the antibody is stronger than the negative dielectrophoretic force (21). On the other hand, a cell (17) of which the antibody on the surface of the cell is not or is weakly bound to the antigen, among the entire population of the mouse spleen cells, is taken out from the micropore because the negative dielectrophoretic force (21) is stronger than the binding force between the antigen and the antibody. That is, mouse spleen cells can be selected by adjusting the strength of the negative dielectrophoretic force to thereby adjust the selection criterion of the binding force between the antibody on the surface of the cell and the antigen. Furthermore, as shown in FIG. 23, the mouse spleen cell taken out from the micropore is captured on the insulating material (8) by disposing a cationic polymer (46) such as polylysine on the insulating material. Next, as shown in FIG. 24, mouse cancer cells (43) are introduced into the cell selection area (1) by re-applying the first AC voltage from the first AC power supply, and, as shown in FIG. 25, the mouse cancer cells are immobilized in the micropores. At this time, as shown in FIG. 25, among the entire population of the mouse spleen cells which have been already taken out from the micropores, a cell (17) of which the antibody on the surface of the cell is not or is weakly bound to the antigen, is captured on the insulating material (8) through the cationic polymer (46), as a result of which this cell is extremely less likely to be re-immobilized in the micropore (9) by dielectrophoresis. That is, mouse cancer cells are brought into contact only with the spleen cells on the surfaces of which antibodies strongly bound to the antigen are presented, and which have been selected by the negative dielectrophoretic force. Thereafter, as shown in FIG. 26, among the entire population of the mouse spleen cells being in contact, cells (18) on the surface of which the antibody is strongly bound to the antigen, are fused with the mouse cancer cells (43) by applying a DC pulse voltage from the DC pulse power supply (35).

The above-mentioned procedure enables the selective fusion between the mouse cancer cells and the mouse spleen cells on the surface of which antibodies strongly bound to an antigen are presented, as a result of which the number of generated fused cells remarkably decreases and the proportion of fused cells capable of producing antibodies that are strongly bindable to the antigen increases. Thus, the cell selection work to pick up fused cells capable of producing antibodies that are strongly bindable to the antigen can be remarkably reduced.

Furthermore, the cell selection method of the present invention may also include the steps of; immobilizing a recognition molecule which can recognize a specific substance of the first cell, onto a surface of the second cell; and binding the first cell to the second cell through a bond between the specific substance and the recognition molecule.
This aspect enables selection of the first cells using both the recognition molecule immobilized on the bottom face of the micropore and the recognition molecule immobilized on the surface of the second cell. Therefore, an effect of further improving the selection efficiency can be given particularly when the cell selection and the cell fusion are conducted in a successive manner in the present invention. In this case, the recognition molecule immobilized on the bottom face of the micropore and the recognition molecule immobilized on the surface of the second cell may be the same recognition molecule which can recognize the same specific substance of the first cell, or different recognition molecules which can respectively recognize two types of different specific substances of the first cell.

If the recognition molecule immobilized on the bottom face of the micropore and the recognition molecule immobilized on the surface of the second cell are the same recognition molecule which can recognize the same specific substance of the first cell, the first cells may be sometimes nonspecifically immobilized in the micropores, or such a case may happen. In the case where the first cells are nonspecifically immobilized in the micropores, or in such a case, they can be again selected by the recognition molecule of the second cell, further improving the accuracy of the cell selection to achieve the fusion between the first cell and the second cell, as a result of which the cell selection efficiency is further improved.

In addition, if the recognition molecule immobilized on the bottom face of the micropore and the recognition molecule immobilized on the surface of the second cell are different recognition molecules which can respectively recognize two types of different specific substances of the first cell, for example, a specific antigen is immobilized on the bottom face of the micropore to select the first cell on the surface of which an antibody strongly bound to the antigen is presented. By immobilizing an anti-IgG antibody which can select the IgG antibody on the surface of the second cell, a first cell on the surface of which a lot of IgG-class antibodies strongly bound to the specific antigen are presented (generally, IgG-class antibodies are used as monoclonal antibodies and IgM-class antibodies are not used) can be exclusively selected. Accordingly, it becomes possible to fuse the first cell and the second cell, as a result of which the cell selection efficiency is further improved. Here, the recognition molecule immobilized on the second cell is not limited to IgG antibodies, and any modification can be made without specific limitations, as long as there is no deviation from the gist of the present invention. For example, besides the above-mentioned example, a specific antigen can be immobilized on the micropore so as to select the first cell on the surface of which an antibody strongly bound to an antigen is presented. Furthermore, if a recognition molecule which can select CD138 presented on the surface of the first cell, is immobilized onto the surface of the second cell, a type of plasma cells which are strongly bound to the specific antigen and which secrete antibodies from themselves can be exclusively selected from the first cells, thereby enabling the cell fusion between the first cell and the second cell.

Furthermore, in the case of this aspect, the micropore and the first cell are immobilized through the bond between the recognition molecule immobilized on the micropore and the specific substance on the surface of the cell, while the first cell and the second cell are contacted through the bond between the specific substance on the surface of the first cell and the recognition molecule immobilized on the surface of the second cell. When applying a specific force acting in a direction to take out the first cell and the second cell from the micropore, a pair is made exclusively between the first cell of which the specific substance on the surface of the cell strongly bound to the recognition molecule immobilized on the micropore is presented, and the second cell of which the recognition molecule on the surface of the cell strongly bound to the specific substance on the surface of the first cell is immobilized, and only this kind of pair is left behind. Accordingly, it becomes possible to fuse these cells by applying the DC pulse voltage while applying the specific force acting in a direction to take out them from the micropore. By so doing, the first cell and the second cell taken out from the micropore under the specific force are always left out of the micropore, as a result of which the first cell and the second cell having a weak bond between the specific substance and the recognition molecule are less likely to be fused, while the first cell and the second cell having a strong bond between the specific substance and the recognition molecule are more likely to be fused, and thereby the selection efficiency of the fused cell can be further improved.

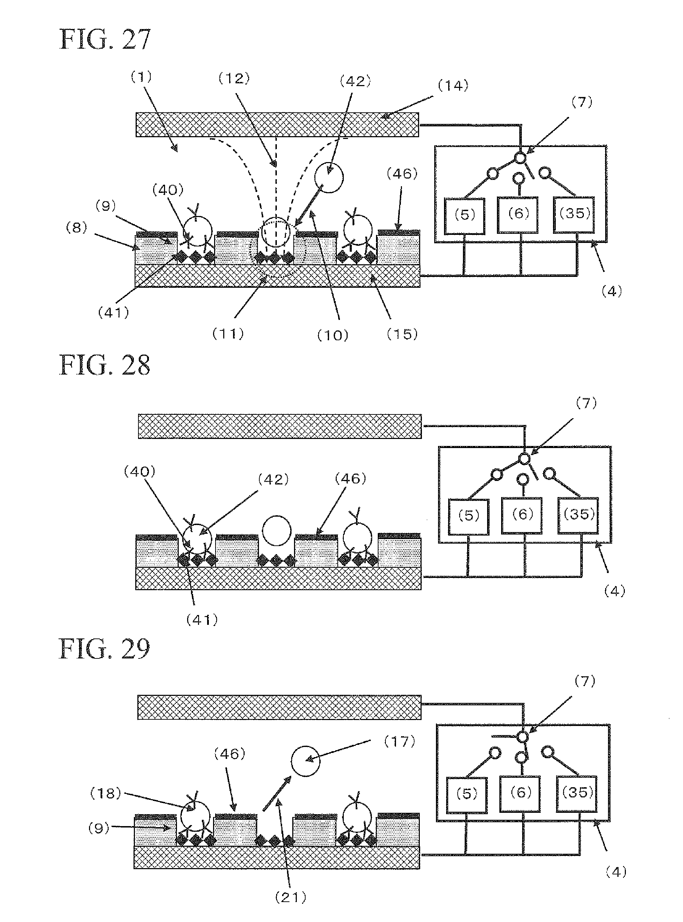

FIG. 27 to FIG. 34 show conceptual diagrams of the method of the present invention where the cell selection and the cell fusion are conducted in a successive manner by applying the DC pulse voltage while applying the specific force acting in a direction to take out cells from the micropore.

In FIG. 27, mouse spleen cells (42) are introduced into a cell selection area (1) where antigens (41) bindable to a specific antibody (40) have been immobilized on bottom faces of micropores, and these mouse spleen cells are immobilized in the micropores (9) by applying a first AC voltage from the first AC power supply (5). Next, as shown in FIG. 28, the mouse spleen cells (42) are brought into contact with the bottom faces of the micropores to thereby cause an antigen-antibody reaction between the antibody (40) presented on the surface of the cell and the antigen (41) immobilized on the bottom face of the micropore, thus making a bond therebetween. Subsequently, as shown in FIG. 29, a negative dielectrophoretic force is effected as the force to take out the mouse spleen cells from the micropores by applying a second AC voltage from the second AC power supply (6). Here, a cell (18) of which the antibody on the surface of the cell is strongly bound to the antigen, among the entire population of the mouse spleen cells immobilized in the micropores, is left behind in the micropore because the binding force between the antigen and the antibody is stronger than the negative dielectrophoretic force. On the other hand, a cell (17) of which the antibody on the surface of the cell is not or is weakly bound to the antigen, among the entire population of the mouse spleen cells, is taken out from the micropore because the negative dielectrophoretic force (21) is stronger than the binding force between the antigen and the antibody. That is, mouse spleen cells can be selected by adjusting the strength of the negative dielectrophoretic force to thereby adjust the selection criterion of the binding force between the antibody on the surface of the cell and the antigen.

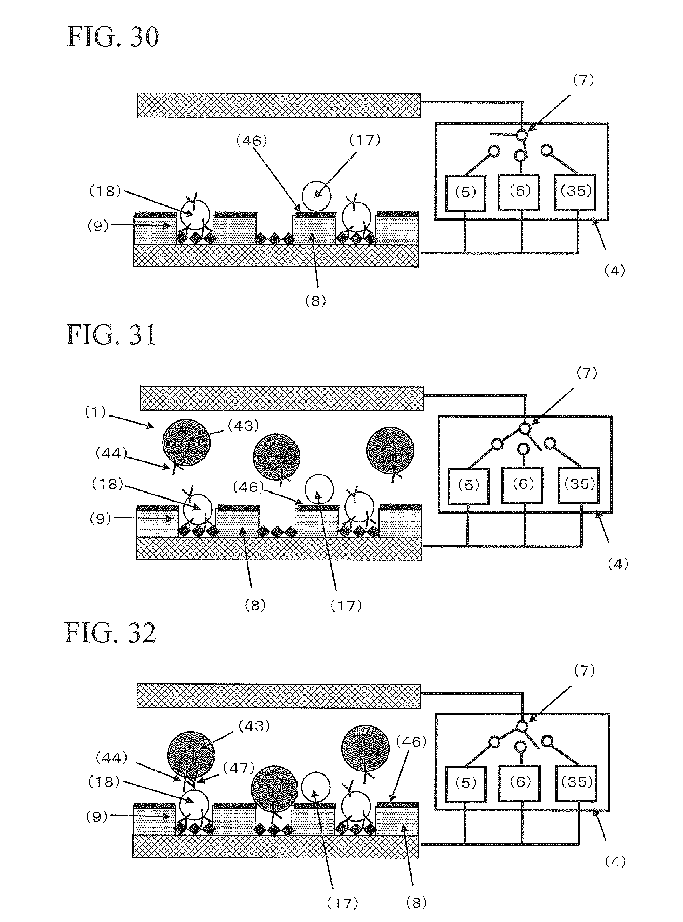

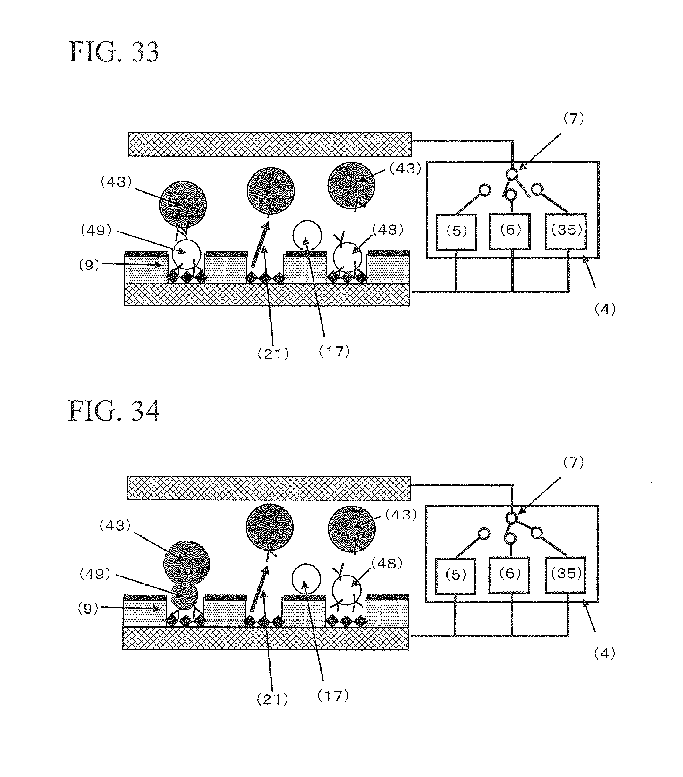

Furthermore, as shown in FIG. 30, the mouse spleen cell taken out from the micropores is captured on the insulating material (8) by disposing a cationic polymer (46) such as polylysine on the insulating material. Next, as shown in FIG. 31, mouse cancer cells (43) on the surfaces of which anti-IgG antibodies (44) are immobilized, are introduced into the cell selection area by re-applying the first AC voltage from the first AC power supply, and as shown in FIG. 32, the mouse cancer cells are immobilized on the micropores. At this time, as shown in FIG. 32, a mouse spleen cell (17) which has been already taken out from the micropore is captured on the insulating material (8) through the cationic polymer (46), as a result of which this cell is extremely less likely to be re-immobilized in the micropore by dielectrophoresis. That is, mouse cancer cells are brought into contact only with the mouse spleen cells on the surfaces of which antibodies strongly bound to the antigen are presented, and which have been selected by the negative dielectrophoretic force. Next, as shown in FIG. 32, among the entire population of the antibodies existing on the surfaces of the mouse spleen cells, an IgG-type antibody (47) and the anti-IgG antibody (44) presented on the surface of the mouse cancer cell are subjected to an antigen-antibody reaction. Next, as shown in FIG. 33, the negative dielectrophoretic force (21) acting in a direction to take out the cells from the micropores (9) is effected by applying a second AC voltage from the second AC power supply (6). At this time, mouse cancer cells (43) are separated from mouse spleen cells (48) presenting a small proportion of IgG-type antibodies on their surface, among the entire population of the mouse spleen cells, by the negative dielectrophoretic force (21); however, a mouse spleen cell (49) presenting a large proportion of IgG-type antibodies on its surface, among the entire population of the mouse spleen cells, is left behind while being contacted with the mouse cancer cell (43). Thereafter, as shown in FIG. 34, the mouse spleen cell and the mouse cancer cell which are in contact with each other are exclusively fused by applying a DC pulse voltage from the DC pulse power supply (35) while applying the second AC voltage from the second AC power supply (6), that is, while applying the negative dielectrophoretic force (21) acting in a direction to take out the cells from the micropores (9).

The above-mentioned procedure enables the selective fusion between the mouse cancer cell and the mouse spleen cell on the surface of which a lot of IgG-type antibodies strongly bound to an antigen are presented, as a result of which the number of generated fused cells remarkably decreases and the proportion of fused cells capable of producing IgG antibodies that are strongly bindable to the antigen increases. Thus, the cell selection work to pick up fused cells capable of producing antibodies that are strongly bindable to the antigen after the cell fusion can be further remarkably reduced.

Here, the method of immobilizing the recognition molecule on the surface of the second cell is not specifically limited as long as the recognition molecule can be immobilized at least on the surface of the second cell. For example, it is possible to effect a physical adsorption by contacting the surface of the second cell and the recognition molecule bindable to the specific substance in a solution which contains the recognition molecule, or it is also possible to utilize a chemical bond such as an amide bond, a biotin-avidin bond, or a thiol bond. Here, a molecule, a protein, or such a substance for facilitating the immobilization of the recognition molecule to the surface of the second cell may be bound to a site of the recognition molecule other than the site at which the specific substance is bound to the recognition molecule, so as to be immobilized on the surface of the second cell. The molecule for facilitating the immobilization of the recognition molecule to the surface of the second cell can be exemplified by biotin, avidin, a thiol group, or a disulfide group, as well as an alkyl chain or a polyethylene glycol chain which contains these groups. Moreover, the protein for facilitating the immobilization of the recognition molecule to the surface of the second cell can be exemplified by BSA, BCP, and KLH.

### EFFECTS OF THE INVENTION

The present invention can offer the following effects.
1) According to the cell selection apparatus and the selection method of the present invention, a single cell can be immobilized per each micropore under the positive dielectrophoretic force from the first AC power supply, and each cell can be taken out from the micropore under the specific force acting in a direction to take out the cell from the micropore so as to be individually selected, making it possible to select a mass of cells on the surfaces of which a specific substance strongly bound to a recognition molecule is presented, all at once easily in a short period of time.

2) According to the cell selection apparatus and the selection method of the present invention, by using the fluid delivery force or the dielectrophoretic force as the specific force, it becomes possible for the first time to easily select cells based on the strength of the binding force between the specific substance on the surface of the cell and the recognition molecule, which has been so far difficult with current techniques.

3) According to the cell selection apparatus and the selection method of the present invention, a first cell immobilized in the micropore can be taken out from the micropore under the positive dielectrophoretic force from the first AC power supply, each cell being taken out from the micropore under the specific force acting in a direction to take out the cell from the micropore so as to be individually selected based on the strength of a binding force between the specific substance on the surface of the cell and the recognition molecule. Moreover, by contacting the second cells with the first cells remaining in the micropores and applying a DC pulse voltage from the DC pulse power supply, first cells on the surfaces of which a specific substance strongly bound to a recognition molecule is presented, can be selectively fused with the second cells. Thus, a mass of cells can be selected and fused in a quite short period of time, based on the evaluation of the binding force between the specific substance on the surface of the cell and the recognition molecule.

4) According to the cell selection apparatus and the selection method of the present invention, a first cell immobilized in the micropore can be taken out from the micropore under the positive dielectrophoretic force from the first AC power supply, each cell being taken out from the micropore under the specific force acting in a direction to take out the cell from the micropore so as to be individually selected based on the strength of a binding force between the specific substance on the surface of the cell and the recognition molecule. Moreover, by contacting the second cell with the first cell remaining in the micropore and applying a DC pulse voltage from the DC pulse power supply, the first cell remaining in the micropore after the cell selection can be forcibly contacted with the second cell by the first AC voltage from the first AC power supply, so that the first cell and the second cell can be fused as a pair. Thus, it becomes possible for the first time to achieve an extremely high refusion probability as compared to conventional cell fusion methods (the PEG method and the electrofusion method), and it becomes substantially possible to generate fused cells after the cell selection.

5) According to the cell selection apparatus and the selection method of the present invention, there is no need of separately preparing an apparatus and a vessel for selecting cells and an apparatus and a vessel for fusing cells, and the cell selection and the cell fusion can be conducted in a successive manner within the same vessel, making it possible to select and fuse a mass of cells easily in a short period of time, to perform a quick and efficient operation as well as to maintain the activity of the cells. Moreover, it also becomes possible to remarkably reduce the loss of cells occurring during the transfer of cells from the vessel for selecting cells into the vessel for fusing cells.

6) According to the cell selection apparatus and the selection method of the present invention, there is no need of an expensive and large-scaled apparatus, and the cell selection and the cell fusion can be conducted in a successive manner in an inexpensive and small-scaled apparatus.

7) According to the cell selection apparatus and the selection method of the present invention, there is no need of any complicated process for separating cells from the support after the cell selection or the cell fusion, and it becomes possible to readily recover the selected cells or the fused cells.

8) According to the cell selection apparatus and the selection method of the present invention, it becomes possible to fuse the first cell and the second cell while applying the specific force acting in a direction to take out these cells from the micropore, as a result of which unnecessary cells taken out from the micropores are less likely to be fused. Thus, fused cells can be selected in a quite short period of time, based on the evaluation of the binding force between the specific substance on the surface of the cell and the recognition molecule.

9) According to the cell selection apparatus and the selection method of the present invention, selective fusion becomes possible between the mouse cancer cell and the mouse spleen cell on the surface of which a lot of IgG-type antibodies strongly bound to an antigen are presented, as a result of which the number of generated fused cells remarkably decreases and the proportion of fused cells capable of producing IgG antibodies that are strongly bindable to the antigen increases, among the entire population of the thus obtained fused cells. Thus, the cell selection work to pick up fused cells capable of producing antibodies that are strongly bindable to the antigen after the cell fusion can be further remarkably reduced.

### BRIEF DESCRIPTION OF THE DRAWINGS

FIG. 1 is a first diagram showing a basic concept of a cell selection method of the present invention.
FIG. 2 is a second diagram showing a basic concept of the cell selection method of the present invention.
FIG. 3 is a third diagram showing a basic concept of the cell selection method of the present invention.
FIG. 4 is a conceptual diagram of the cell selection apparatus of the present invention, as well as being a conceptual diagram of the cell selection apparatus used in Examples 1 and 2.
FIG. 5 is a cross-sectional view taken along the line XX' of FIG. 4.
FIG. 6 shows an example of micropores for use in the present invention, in a case where the diameter of a maximum circle inscribed in the planar shape of the micropore is larger than twice the diameter of a cell to be immobilized on the micropore.
FIG. 7 shows an example of micropores for use in the present invention, in a case where the diameter of a maximum circle inscribed in the planar shape of the micropore is smaller than the diameter of a cell to be immobilized on the micropore.
FIG. 8 shows an example of micropores for use in the present invention, in a case where the diameter of a maximum circle inscribed in the planar shape of the micropore is the same to twice the diameter of a cell to be immobilized on the micropore, and where the depth of the micropore is the same or smaller than the diameter of the cell to be immobilized on the micropore.
FIG. 9 shows an example of the waveform of an AC voltage for use in the present invention, where a typical waveform of a sine wave is shown, the horizontal axis (X-axis) representing the time and the vertical axis (Y-axis) representing the voltage.
FIG. 10 shows an example of the waveform of an AC voltage for use in the present invention, where a typical waveform of a triangular wave is shown, the horizontal axis (X-axis) representing the time and the vertical axis (Y-axis) representing the voltage.
FIG. 11 shows an example of the waveform of an AC voltage for use in the present invention, where a typical waveform of a trapezoid wave is shown, the horizontal axis (X-axis) representing the time and the vertical axis (Y-axis) representing the voltage.
FIG. 12 shows an example of the waveform of an AC voltage for use in the present invention, where a typical waveform of a rectangular wave is shown, the horizontal axis (X-axis) representing the time and the vertical axis (Y-axis) representing the voltage.
FIG. 13 is a diagram showing a concept of the method for choosing the strength of the binding force between the specific substance on the surface of the cell and the recognition molecule of the present invention based on the strength of the negative dielectrophoretic force.
FIG. 14 is a schematic diagram showing a typical photolithography and etching method for producing the micropored insulating material-integrated lower electrode used in the Examples.
FIG. 15 is a graph showing relations of the retention rate of antibody-immobilized polystyrene beads in micropores relative to the concentration of immobilized antibodies, with the passage of time as shown in Example 1, the horizontal axis (X-axis) representing the applied voltage (indicated by volt (V)) and the vertical axis (Y-axis) representing the bead retention rate (%).
FIG. 16 is a graph showing relations of the retention rate of antibody-immobilized polystyrene beads in micropores relative to the concentration of immobilized antibodies, with the application of voltage as shown in Example 2, the horizontal axis (X-axis) representing the applied voltage (indicated by volt(V)) and the vertical axis (Y-axis) representing the bead retention rate (%).
FIG. 17 is a conceptual diagram of a case where a gravitational force is effected on a cell in a direction to take out the cell from the micropore in the present invention.
FIG. 18 is a conceptual diagram of a case where a magnetic force is effected on a cell in a direction to take out the cell from the micropore in the present invention.
FIG. 19 is a conceptual diagram of a case where a fluid delivery force is effected on a cell in a direction to take out the cell from the micropore in the present invention.
FIG. 20 is a first diagram showing a concept of the cell selection method of the present invention where the cell selection and the cell fusion are conducted in a successive manner.
FIG. 21 is a second diagram showing a concept of the cell selection method of the present invention where the cell selection and the cell fusion are conducted in a successive manner.
FIG. 22 is a third diagram showing a concept of the cell selection method of the present invention where the cell selection and the cell fusion are conducted in a successive manner.
FIG. 23 is a fourth diagram showing a concept of the cell selection method of the present invention where the cell selection and the cell fusion are conducted in a successive manner.
FIG. 24 is a fifth diagram showing a concept of the cell selection method of the present invention where the cell selection and the cell fusion are conducted in a successive manner.
FIG. 25 is a sixth diagram showing a concept of the cell selection method of the present invention where the cell selection and the cell fusion are conducted in a successive manner.
FIG. 26 is a seventh diagram showing a concept of the cell selection method of the present invention where the cell selection and the cell fusion are conducted in a successive manner.
FIG. 27 is a first diagram showing a concept of the cell selection method of the present invention where the cell selection and the cell fusion are conducted in a successive manner, in a case where the cell fusion is conducted while applying the specific force acting in a direction to take out the cell from the micropore.
FIG. 28 is a second diagram showing a concept of the cell selection method of the present invention where the cell selection and the cell fusion are conducted in a successive manner, in a case where the cell fusion is conducted while applying the specific force acting in a direction to take out the cell from the micropore.
FIG. 29 is a third diagram showing a concept of the cell selection method of the present invention where the cell selection and the cell fusion are conducted in a successive manner, in a case where the cell fusion is conducted while applying the specific force acting in a direction to take out the cell from the micropore.
FIG. 30 is a fourth diagram showing a concept of the cell selection method of the present invention where the cell selection and the cell fusion are conducted in a successive manner, in a case where the cell fusion is conducted while applying the specific force acting in a direction to take out the cell from the micropore.
FIG. 31 is a fifth diagram showing a concept of the cell selection method of the present invention where the cell selection and the cell fusion are conducted in a successive manner, in a case where the cell fusion is conducted while applying the specific force acting in a direction to take out the cell from the micropore.
FIG. 32 is a sixth diagram showing a concept of the cell selection method of the present invention where the cell selection and the cell fusion are conducted in a successive manner, in a case where the cell fusion is conducted while applying the specific force acting in a direction to take out the cell from the micropore.
FIG. 33 is a seventh diagram showing a concept of the cell selection method of the present invention where the cell selection and the cell fusion are conducted in a successive manner, in a case where the cell fusion is conducted while applying the specific force acting in a direction to take out the cell from the micropore.
FIG. 34 is an eighth diagram showing a concept of the cell selection method of the present invention where the cell selection and the cell fusion are conducted in a successive manner, in a case where the cell fusion is conducted while applying the specific force acting in a direction to take out the cell from the micropore.

### EMBODIMENTS FOR CARRYING OUT THE INVENTION

Hereunder is a detailed description of embodiments of the present invention. The present invention is not to be limited to these Examples and any modification can be optionally made without departing from the gist of the present invention.

### EXAMPLES

### (Example 1)

FIG. 4 is a conceptual diagram of the cell selection apparatus used in Example 1. The cell selection apparatus is roughly composed of a cell selection vessel (13) and a power supply (4). As shown in FIG. 4, the cell selection vessel has a structure in which a spacer (16) is disposed between an upper electrode (14) and a lower electrode (15), and an insulating material (8) having a plurality of micropores formed in an array like arrangement is interposed between the spacer and the lower electrode. As will be described later, the micropores have been formed in the insulating material disposed on the lower electrode (15) by usual photolithography and etching.

For the upper electrode and the lower electrode, a Pyrex (registered trademark) substrate in a length of 70 mm, a width of 40 mm, and a thickness of 1 mm formed with an ITO film (thickness of 150 nm) was used. For the spacer, a silicone sheet in a length of 40 mm, a width of 40 mm, and a thickness of 1.5 mm in the center of which a hollow was made in a length of 20 mm and a width of 20 mm was used. In addition, as shown in FIG. 4, the spacer is provided with an inlet channel (29) for introducing cells, an inlet port (19) communicated therewith, an outlet channel (30) for discharging cells, and an outlet port (20) communicated therewith, so as to introduce/discharge cells into/out from the cell selection vessel.
Moreover, the insulating material (8) having a plurality of micropores was produced by integrally forming with the lower electrode by the photolithography and etching method shown in FIG. 14.

First, the ITO film surface of the Pyrex (registered trademark) glass (24) which has been formed with ITO (23) was coated with a resist (25) in a thickness of 5 µm using a spin coater, was subjected to natural drying for 45 minutes, and then was prebaked using a hot plate (at 80°C for 15 minutes). For the resist, a xylene-based negative type resist was used. Next, the resist was exposed (27) with an UV exposure device by using an exposure photomask (26) printed with a pattern of micropores having a diameter φ of 8.5 µm in an array like arrangement in which lengthwise 1500 micropores and widthwise 1500 micropores are arranged at lengthwise and widthwise intervals of 20 µm in an area having a length of 30 mm and a width of 30 mm. Then, this resist was developed with a developing solution (32). The exposure time and the development time were adjusted so that the depth of the micropores was equal to the thickness of the resist at 5 µm, to thereby expose the ITO on the bottom faces of the micropores. After the development, the resist was cured by postbaking using a hot plate (at 115°C for 30 minutes).

The thus produced upper electrode (14), spacer (16), and micropored insulating material-integrated lower electrode (28) were stacked as shown in FIG. 5 and attached by pressure. FIG. 5 is a cross-sectional view of the cell selection vessel taken along the line XX' of FIG. 4. Since the surface of the silicone sheet serving as the spacer was sticky, the respective parts were adhered with each other, thus enabling a cell fusion solution A containing cells to be charged into the cell selection vessel without leaking. Since the dimension of the hollow in the spacer had a length of 20 mm and a width of 20 mm, the number of micropores in this space was about one million.

In addition, on the lower electrode on the bottom faces of the micropores were immobilized an E2-BSA antigen produced by binding estradiol (E2) to bovine serum albumin (BSA) through the following treatment. That is, the micropored insulating material-integrated lower electrode was soaked in an E2-BSA antigen solution (2.0 µg/mL) for about 12 hours, to effect a physical adsorption of the E2-BSA antigen to the lower electrode on the bottom faces of the micropores, and then was washed with pure water. Next, for the purpose of blocking to prevent nonspecific adsorption of antibodies, the micropored insulating material-integrated lower electrode was soaked in a skim milk 1% PBS solution (containing 0.01 weight % NaNO₃) for about 12 hours, to effect blocking of the lower electrode on the bottom faces of the micropores with skim milk, and then was washed with pure water. This treatment brings physical adsorption and immobilization of the E2-BSA antigen and skim milk components onto the insulating material which constitutes the micropores. However, in the actual operation, as will be described later, since the antigen-antibody reaction was conducted after polystyrene beads were placed in the micropores, the immobilization of the E2-BSA antigen on the surface of the insulating material would not cause a big influence on this Example.

Moreover, the power supply (4) for applying a voltage between electrodes includes a first AC power supply (5) for applying a first AC voltage, a second AC power supply (6) for applying a second AC voltage, and a cell-fusion power supply (35) for applying a DC pulse voltage, between the upper electrode and the lower electrode. The first AC power supply, the second AC power supply, and the cell-fusion power supply can be used by appropriately switching over by the power supply-switchover mechanism (7). The first power supply serves as a cell-immobilization power supply, and the second power supply serves as a cell-taking power supply. In this Example, a WF1966 manufactured by NF Corporation was used for the first AC power supply and the second AC power supply, and an LF101 manufactured by NEPA GENE Co., Ltd. was used for the cell-fusion power supply.

The cell selection apparatus including the micropored insulating material-integrated lower electrode mentioned above was used to perform experiments that will be described later. As a model system of the cell selection, polystyrene beads (Polybead (registered trademark) Polystyrene Microspheres manufactured by Polysciences Inc.) having a diameter of 6.0 µm were used this time.

The polystyrene beads were immobilized with mouse anti-E2 antibodies by contacting a mouse anti-E2 antibody solution at respective concentrations of 0, 0.1, 1.0, and 2.0 µg/mL to the surfaces of the polystyrene beads to effect a physical adsorption, and then were blocked. The immobilization and blocking method with the mouse anti-E2 antibodies was the same as the immobilization and blocking method of the lower electrode on the bottom faces of the micropores with the E2-BSA antigen mentioned above. Here, polystyrene beads contacted with a higher concentration of mouse anti-E2 antibody solution are assumed to have a greater number of mouse anti-E2 antibodies immobilized on the surfaces of the polystyrene beads. Hereunder, these polystyrene beads immobilized with the mouse anti-E2 antibodies on their surfaces are referred to as antibody-immobilized polystyrene beads.

600 µL of the suspension of the antibody-immobilized polystyrene beads made by immobilization of the antibodies at each antibody concentration (bead concentration: about 1.65 × 10⁶ beads/mL) was respectively injected from the inlet port of the spacer into the cell selection area by using a 1 mL volume dispenser. For example, the number of antibody-immobilized polystyrene beads introduced into the cell selection area was about 9.91 × 10⁵, when using a mouse anti-E2 antibody solution at a concentration of 2.0 µg/mL, in which case the number of the introduced antibody-immobilized polystyrene beads was approximately the same as the number of micropores, about one million, in the cell selection area. Then, by leaving it still for about 5 minutes, the antibody-immobilized polystyrene beads were entered into about 37% of the micropores in the cell selection area due to gravitational sedimentation. That is, the number of antibody-immobilized polystyrene beads which finally entered the micropores was about 3.68 × 10⁵. The other antibody-immobilized polystyrene beads remained on the insulating material between the micropores.

Here, in this Example 1 and Example 2 that will be described later, the retention rate was calculated by the following equation, on the basis of the number of antibody-immobilized polystyrene beads which finally remained in the micropores when introducing antibody-immobilized polystyrene beads at each concentration of 0, 0.1, 1.0, and 2.0 µg/mL into the cell selection area (that is, the number of antibody-immobilized polystyrene beads which were originally inside the micropores) (retention rate = number of antibody-immobilized polystyrene beads held in the micropores/number of antibody-immobilized polystyrene beads which were originally inside the micropores × 100).

The number of antibody-immobilized polystyrene beads which had entered the micropores was obtained by counting the number of antibody-immobilized polystyrene beads held in the micropores in the region of one-tenth of the cell selection area (any 6 mm × 6 mm region in the cell selection area, and about 0.1 million micropores exist in this region), and multiplying the counted number by ten (this procedure is the same in Example 2).

Next, by leaving it still at room temperature for about 40 minutes, the antibody-immobilized polystyrene beads inside the micropores were contacted with the bottom faces of the micropores to effect the antigen-antibody reaction between the antibody on the surface of the antibody-immobilized polystyrene bead and the antigen immobilized on the bottom face of the micropore. Subsequently, an AC voltage having a frequency of 3 MHz and a voltage of 2.5, 5.0, 7.5, 10, or 15 Vpp respectively was applied between electrodes by the second AC power supply. One minute later, the movement of these antibody-immobilized polystyrene beads was observed with the passage of time. Unlike cells, the polystyrene beads are affected by a negative dielectrophoretic force when the frequency of the second AC voltage is high (for example, 1 MHz to 3 MHz), and affected by a positive dielectrophoretic force when the frequency of the second AC voltage is low (for example, 1 kHz to 10 kHz).

At this time, in the case of antibody-immobilized polystyrene beads without an immobilization of any antibodies (antibody-immobilized polystyrene beads treated with a mouse anti-E2 antibody solution having zero concentration of mouse anti-E2 antibodies), almost all antibody-immobilized polystyrene beads, among the entire population of the antibody-immobilized polystyrene beads which were originally inside the micropores, were detached from the micropores as the time went by. One minute after the application of a voltage of 7.5 V, only 0.7% antibody-immobilized polystyrene beads (about 2.42 × 10³ beads), among the entire population of the antibody-immobilized polystyrene beads which were originally inside the micropores (about 3.45 × 10⁵ beads), were held in the micropores.

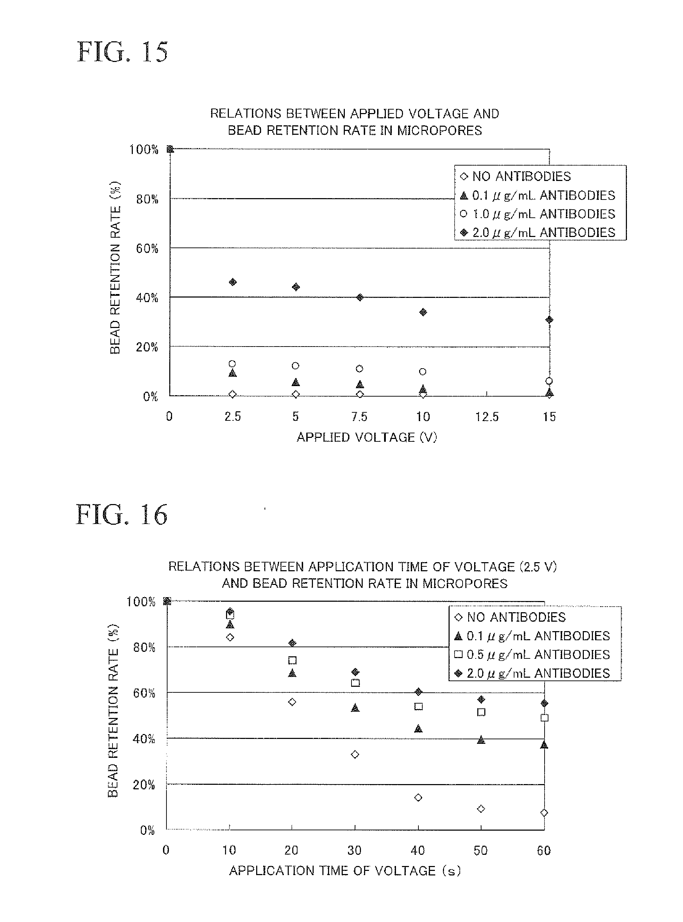

Moreover, in the case of antibody-immobilized polystyrene beads treated with a mouse anti-E2 antibody solution having a relatively low concentration of mouse anti-E2 antibodies at 0.1 µg/mL, about 5.0% antibody-immobilized polystyrene beads (about 1.56 × 10⁴ beads), among the entire population of the antibody-immobilized polystyrene beads which were originally inside the micropores (about 3.12 × 10⁵ beads), were held in the micropores, one minute after the application of a voltage of 7.5 V. As shown in FIG. 15, the proportion of antibody-immobilized polystyrene beads detached from the micropores increased as the applied voltage increased.

Furthermore, in the case of antibody-immobilized polystyrene beads treated with a mouse anti-E2 antibody solution having a relatively high concentration of mouse anti-E2 antibodies at 2.0 µg/mL, 40% antibody-immobilized polystyrene beads (about 1.47 × 10⁵ beads), among the entire population of the antibody-immobilized polystyrene beads which were originally inside the micropores (about 3.68 × 10⁵ beads), were held in the micropores, one minute after the application of a voltage of 7.5 V. As shown in FIG. 15, the proportion of antibody-immobilized polystyrene beads detached from the micropores increased as the applied voltage increased.

From these results, because it can be considered that a greater number of mouse anti-E2 antibodies immobilized on the surfaces of the antibody-immobilized polystyrene beads leads to a stronger binding force to the antigen on the bottom face of the micropore, the strength of the binding force between the antibodies immobilized on the surfaces of the antibody-immobilized polystyrene beads and the antigens immobilized on the bottom faces of the micropores was able to be chosen, based on the magnitude of the voltage of the second AC power supply, easily and within a short period of time of about 30 seconds.

### (Example 2)

600 µL of the suspension of the antibody-immobilized polystyrene beads whose surfaces were immobilized with anti-E2 antibodies, made by using the mouse anti-E2 antibody solution at respective concentrations of 0, 0.1, 0.5, and 2.0 µg/mL of Example 1 (bead concentration: about 1.65 × 10⁶ beads/mL), was respectively introduced from the inlet port of the spacer into the cell selection area by using a 1 mL volume dispenser. Then, by leaving it still for about 5 minutes, the antibody-immobilized polystyrene beads were subjected to gravitational sedimentation. Furthermore, by leaving it still at room temperature for about 40 minutes, the antibody-immobilized polystyrene beads inside the micropores were contacted with the bottom faces of the micropores to effect the antigen-antibody reaction between the antibody on the surface of the antibody-immobilized polystyrene bead and the antigen immobilized on the bottom face of the micropore. Subsequently, an AC voltage of 2.5 Vpp having a frequency of 3 MHz was applied between electrodes by the second AC power supply, and the movement of these antibody-immobilized polystyrene beads was observed with the passage of time.

At this time, in the case of antibody-immobilized polystyrene beads without an immobilization of any antibodies (antibody-immobilized polystyrene beads treated with a mouse anti-E2 antibody solution having zero concentration of mouse anti-E2 antibodies), many antibody-immobilized polystyrene beads, among the entire population of the antibody-immobilized polystyrene beads which were originally inside the micropores, were detached from the micropores as the time went by. One minute later, 7.6% antibody-immobilized polystyrene beads (about 2.52 × 10⁴ beads), among the entire population of the antibody-immobilized polystyrene beads which were originally inside the micropores (about 3.32 × 10⁵ beads), were held in the micropores. As shown in FIG. 16, the proportion of antibody-immobilized polystyrene beads detached from the micropores increased as the voltage application time went by.

Moreover, in the case of antibody-immobilized polystyrene beads treated with a mouse anti-E2 antibody solution having a relatively low concentration of mouse anti-E2 antibodies at 0.1 µg/mL, 37.5% antibody-immobilized polystyrene beads (about 1.28 × 10⁵ beads), among the entire population of the antibody-immobilized polystyrene beads which were originally inside the micropores (about 3.41 × 10⁵ beads), were held in the micropores, one minute later. As shown in FIG. 16, the proportion of antibody-immobilized polystyrene beads detached from the micropores increased as the voltage application time went by.

Furthermore, in the case of antibody-immobilized polystyrene beads treated with a mouse anti-E2 antibody solution having a relatively high concentration of mouse anti-E2 antibodies at 2.0 µg/mL, 55.6% antibody-immobilized polystyrene beads (about 2.13 × 10⁵ beads), among the entire population of the antibody-immobilized polystyrene beads which were originally inside the micropores (about 3.83 × 10⁵ beads), were held in the micropores, one minute later. As shown in FIG. 16, the proportion of antibody-immobilized polystyrene beads detached from the micropores increased as the voltage application time went by.

From these results, similarly to Example 1, because it can be considered that a greater number of mouse anti-E2 antibodies immobilized on the surfaces of the antibody-immobilized polystyrene beads leads to a stronger binding force to the antigen on the bottom face of the micropore, the strength of the binding force between the antibodies immobilized on the surfaces of the antibody-immobilized polystyrene beads and the antigens immobilized on the bottom faces of the micropores was able to be chosen, based on the voltage application time of the second AC power supply, easily and within a short period of time of about 30 seconds.

### (Example 3)

Using the same cell selection apparatus of Example 1, spleen cells presenting a specific antibody on their surfaces were selected. Regarding the cells, non-immunized mouse spleen A cells and mouse spleen B cells immunized with an E2 antigen were used. In the mouse spleen B cells immunized with an E2 antigen, there exist cells presenting the E2 antibody on their surfaces (hereunder, referred to as antibody-presenting cells). This Example 3 shows an example in which antibody-presenting cells are selected by identifying the binding force of the antigen-antibody reaction between the antibody presented by this antibody-presenting cell and the antigen immobilized on the bottom face of the micropore, based on the magnitude of the AC voltage of the second AC power supply which defines the strength of the negative dielectrophoretic force.

First, the mouse spleen A cells and the mouse spleen B cells were respectively suspended in a 300 mM mannitol aqueous solution, and each cell suspension was adjusted to a density of 0.7 × 10⁶ cells/mL.

Next, 600 µL of the cell suspension containing the mouse spleen A cells was injected from the inlet port of the spacer by using a 1 mL volume dispenser. The number of mouse spleen A cells contained in this cell suspension was counted and was found to be about 4.11 × 10⁵. A rectangular wave AC voltage of 10 Vpp having a frequency of 3 MHz was applied between the electrodes by the first AC power supply, as a result of which almost a single mouse spleen A cell per each micropore was able to be immobilized in about 38% of the micropores formed in the array like arrangement, in a quite short period of time of about 2 to 3 seconds. That is, the number of mouse spleen A cells immobilized on the micropores was about 3.80 × 10⁵.

Similarly to Example 1, the number of spleen cells immobilized on the micropores was obtained by counting the number of spleen cells held in the micropores in the region of one-tenth of the cell selection area (any 6 mm × 6 mm region in the cell selection area, and about 0.1 million micropores exist in this region), and multiplying the counted number by ten (hereunder, the number of spleen cells immobilized on the micropores was obtained in the same manner in this Example).

Next, the mouse spleen A cells were contacted with the bottom faces of the micropores, and left still at room temperature for about 40 minutes. During this period, if the E2 antibody was presented on the surface of the mouse spleen A cell, the antibody would bind to the E2 antigen immobilized on the bottom face of the micropore through an antigen-antibody reaction; while, if the E2 antibody was not presented on the surface of the mouse spleen A cell, the antibody would not bind to the E2 antigen immobilized on the bottom face of the micropore without an occurrence of an antigen-antibody reaction. Subsequently, an AC voltage of 5 Vpp having a frequency of 10 kHz was applied between electrodes by the second AC power supply for 30 seconds, to effect a negative dielectrophoretic force. As a result, the movement of almost all mouse spleen A cells being detached from the micropores was observed. Next, 700 µL of a 300 mM mannitol aqueous solution was injected from the inlet port of the spacer by using a 1 mL volume dispenser. These mouse spleen A cells detached from the micropores were discharged from the outlet port. Then, the number of cells left in the micropores was counted and was found to be about 8.2 × 10². That is, about 0.2% (= 8.2 × 10²/3.80 × 10⁵ × 100) mouse spleen A cells, among the entire population of the mouse spleen A cells introduced into the cell selection apparatus, were held in the micropores. This can be assumed because almost all mouse spleen A cells were detached from the micropores because there was almost no existence of antibody-presenting cells presenting the E2 antibody on their surfaces, in the non-immunized mouse spleen A cells, causing no antigen-antibody reaction with the E2 antigen immobilized on the bottom faces of the micropores.

Next, 600 µL of the cell suspension containing the mouse spleen B cells was injected from the inlet port of the spacer by using a 1 mL volume dispenser. The number of mouse spleen B cells contained in this cell suspension was counted and was found to be about 3.98 × 10⁵. A rectangular wave AC voltage of 10 Vpp having a frequency of 3 MHz was applied between the electrodes by the first AC power supply, as a result of which almost a single mouse spleen A cell per each micropore was able to be immobilized in about 36% of the micropores formed in the array like arrangement, in a quite short period of time of about 2 to 3 seconds. That is, the number of mouse spleen A cells immobilized on the micropores was about 3.62 × 10⁵.

Next, the mouse spleen B cells were contacted with the bottom faces of the micropores, and left still at room temperature for about 40 minutes. During this period, if the anti-E2 antibody was presented on the surface of the mouse spleen B cell, the antibody would bind to the E2 antigen immobilized on the bottom face of the micropore through an antigen-antibody reaction; while, if the E2 antibody was not presented on the surface of the mouse spleen B cell, the antibody would not bind to the E2 antigen immobilized on the bottom face of the micropore without an occurrence of an antigen-antibody reaction. Subsequently, an AC voltage of 5 Vpp having a frequency of 10 kHz was applied between electrodes by the second AC power supply for 30 seconds, to effect a negative dielectrophoretic force. As a result, the movement of some mouse spleen B cells being detached from the micropores was observed. Next, 700 µL of a 300 mM mannitol aqueous solution was injected from the inlet port of the spacer by using a 1 mL volume dispenser. These mouse spleen B cells detached from the micropores were discharged from the outlet port. Then, the number of cells left in the micropores was counted and was found to be about 1.20 × 10⁴. That is, about 3.3% (= 1.20 × 10⁴/3.62 × 10⁵ × 100) mouse spleen B cells, among the entire population of the mouse spleen B cells introduced into the cell selection apparatus, were held in the micropores. This can be assumed because there existed about 3.3% mouse spleen B cells bound through a strong antigen-antibody reaction which were not able to be freed by the negative dielectrophoretic force with a voltage of 5 Vpp and a frequency of 10 kHz.

Next, similarly, 600 µL of the cell suspension containing the mouse spleen B cells was injected from the inlet port of the spacer by using a 1 mL volume dispenser. The number of mouse spleen B cells contained in this cell suspension was counted and was found to be about 4.18 × 10⁵. A rectangular wave AC voltage of 10 Vpp having a frequency of 3 MHz was applied between the electrodes by the first AC power supply, as a result of which almost a single mouse spleen A cell per each micropore was able to be immobilized in about 39% of the micropores formed in the array like arrangement, in a quite short period of time of about 2 to 3 seconds. That is, the number of mouse spleen A cells immobilized on the micropores was about 3.89 × 10⁵.

Next, the mouse spleen B cells were contacted with the bottom faces of the micropores, and left still at room temperature for about 40 minutes. During this period, if the E2 antibody was presented on the surface of the mouse spleen B cell, the antibody would bind to the E2 antigen immobilized on the bottom face of the micropore through an antigen-antibody reaction; while, if the E2 antibody was not presented on the surface of the mouse spleen B cell, the antibody would not bind to the E2 antigen immobilized on the bottom face of the micropore without an occurrence of an antigen-antibody reaction. Subsequently, an AC voltage of 8 Vpp having a frequency of 10 kHz was applied between electrodes by the second AC power supply for 30 seconds, to effect a negative dielectrophoretic force. As a result, the movement of some mouse spleen B cells being detached from the micropores was observed. Next, 700 µL of a 300 mM mannitol aqueous solution was injected from the inlet port of the spacer by using a 1 mL volume dispenser. These mouse spleen B cells detached from the micropores were discharged from the outlet port. Then, the number of cells left in the micropores was counted and was found to be about 4.67 × 10³. That is, about 1.2% (= 4.67 × 10³/3.89 × 10⁵ × 100) mouse spleen B cells, among the entire population of the mouse spleen B cells introduced into the cell selection apparatus, were held in the micropores. This can be assumed because there existed about 1.2% mouse spleen B cells bound through an antigen-antibody reaction which was stronger than the negative dielectrophoretic force with a voltage of 8 Vpp and a frequency of 10 kHz.

From the above-mentioned results, it was confirmed that cells presenting an antibody on their surfaces were selected by identifying the binding force between the antibody presented on the surface and the antigen, based on the magnitude of the AC voltage from the second AC power supply which generates the negative dielectrophoretic force.

### (Comparative Example 1)

Similarly to Example 3, spleen cells presenting a specific antibody on their surfaces were selected. Regarding the cells, non-immunized mouse spleen A cells and mouse spleen B cells immunized with an E2 antigen were used. This Comparative Example shows an example in which mouse spleen cells presenting the E2 antibody on their surfaces, are selected by: subjecting antibody-presenting cells existing in the mouse spleen A cells and the mouse spleen B cells to an antigen-antibody reaction with biotinated E2-BSA, and a subsequent reaction (biotin-avidin bond) with streptavidin magnetic beads (µMACS streptavidin kit manufactured by MACS corporation) to thereby selectively bind the antibody-presenting cells to the magnetic beads; and selectively collecting the magnetic beads bound to the antibody-presenting cells by using a magnet.

Similarly to Example 3, non-immunized mouse spleen A cells and mouse spleen B cells immunized with an E2 antigen were respectively suspended in a buffer solution (µMACS streptavidin kit manufactured by MACS corporation), and each cell suspension was adjusted to a density of 2.0 × 10⁸ cells/mL. Next, the adjusted spleen cells were respectively mixed with a biotinated E2-BSA solution to effect an antigen-antibody reaction. The number of mouse spleen A cells mixed with the biotinated E2-BSA solution at this time was about 1.31 × 10⁸, and the number of mouse spleen B cells thereof was about 1.55 × 10⁸. During this period, if the E2 antibody was presented on the surfaces of the mouse spleen cells, the antibody would bind to the E2 antigen of the biotinated E2-BSA through an antigen-antibody reaction; while, if the E2 antibody was not presented on the surfaces of the mouse spleen cells, the antibody would not bind to the E2 antigen of the biotinated E2-BSA without an occurrence of an antigen-antibody reaction.

Subsequently, the mouse spleen cells biotinated through the antigen-antibody reaction were mixed with streptavidin magnetic beads to effect a biotin-avidin bond. Next, the magnetic beads were isolated from the cell suspension by using a magnet. Thereafter, the number of mouse spleen cells collected with the magnetic beads was counted, by which the number of mouse spleen A cells was found to be about 3.91 × 10⁵ and the number of mouse spleen B cells was found to be about 4.95 × 10⁶. The number of mouse spleen cells collected with the magnetic beads was obtained such that the cell suspension in which the mouse spleen cells collected with the magnetic beads were suspended with a buffer solution (µMACS streptavidin kit manufactured by MACS corporation) was added dropwise onto a hemocytometer, and the number of these cells was counted.

From these results, in the case of the above-mentioned mouse spleen A cells, about 0.3% mouse spleen A cells (= 3.91 × 10⁵/1.31 × 10⁸ × 100 cells) were held on the magnetic beads. This can be assumed because almost all mouse spleen A cells were unable to be collected by the magnetic beads because there was almost no existence of antibody-presenting cells presenting the anti-E2 antibody on their surfaces, in the non-immunized mouse spleen A cells, causing no antigen-antibody reaction with the biotinated E2 antigen, accordingly causing no biotin-avidin bond with the streptavidin magnetic bead.

Moreover, in the case of the above-mentioned mouse spleen B cells, about 3.2% mouse spleen B cells (= 4.95 × 10⁶/1.55 × 10⁸ × 100 cells) were held on the magnetic beads. This can be assumed because there existed about 3.2% mouse spleen B cells bound with the biotinated E2 antigen through an antigen-antibody reaction.
In this way, in this Comparative Example, the mouse spleen cells were able to be selected based on whether or not they were bound to the magnetic beads; however, these spleen cells were not able to be selected based on the binding force between the antigen immobilized on the magnetic bead and the antibody presented on the surface of the spleen cell.

### (Example 4)

Using the same cell selection apparatus of Example 1, mouse spleen cells (diameter of about 6 µm), and mouse cancer cells (diameter of about 10 µm), a comparison was made between the case where the cell fusion was performed without performing the selection of mouse spleen cells by using micropores, and the case where the cell fusion was performed after performing the selection of mouse spleen cells by using micropores. The experimental procedure is shown below. All laboratory instruments, reagents, and components of the cell selection vessels were sterilized. The cell selection apparatus to perform the cell fusion without performing the selection of mouse spleen cells by using micropores was the cell selection apparatus of Example 1 in which no E2-BSA antigen was immobilized on the lower electrode on the bottom faces of the micropores.

The mouse spleen cells were produced by immunizing a mouse with an E2-BCP (BCP: Blue Carrier Immunogenic Protein) complex by intraperitoneal injection repeatedly more than ten times at every two weeks. Hereunder is a description of the procedure for taking out spleen cells from the mouse.

First, a mouse was euthanatized by placing a Kimtowel with Sevoflurane (manufactured by Maruishi Pharmaceutical Co., Ltd.) in a hermetic bottle. The mouse was sufficiently sprayed with 70% ethanol for disinfection, and then immobilized on an autopsy table with injection needles in a clean bench. Next, the outer skin was lifted up with tweezers and cut in with dissecting scissors to cut off the outer skin first. Next, the subendothelial layer was incised with new different scissors to expose the spleen. The spleen was resected from the body of the mouse with the scissors while withdrawing the spleen from the body with the tweezers. Into a 50 mL centrifugal tube was charged 10 mL of an animal cell culture medium containing 10% FBS (bovine serum) (hereunder, referred to as a medium A). The spleen was transferred therein and was shaken to wash the surface. Next, the spleen was transferred on a lid of a φ 9 cm Petri dish manufactured by Sumiron, and fat adhered around the resected spleen was removed with two sets of tweezers. Into the φ 9 cm Petri dish manufactured by Sumiron was charged 10 mL of the medium A. The spleen was cut into four to five pieces with new scissors inside a 40 mL mesh cell strainer (manufactured by Falcon), and the spleen was sufficiently mashed with a flat tail of a 5 mL Terumo syringe. The spleen cells adhered to the cell strainer and the tail of the Terumo syringe were rinsed off with the medium A. The spleen cell-containing suspension inside the Petri dish was transferred into a 50 mL centrifugal tube. The Petri dish was rinsed with the medium A twice, and the wash liquid was also mixed in the centrifugal tube. The spleen cell-containing suspension was centrifuged at 1500 rpm at room temperature for 5 minutes, and then the supernatant was drawn by an aspirator. The spleen cell pellet was loosened. Next, the loosened spleen cells were suspended in 1 mL of FBS, added with 9 mL of a red blood cell lysing solution (manufactured by SIGMA), well mixed, and left still at room temperature for 3 minutes to lyse the red blood cells. The spleen cell-containing suspension was again centrifuged at 1500 rpm at room temperature for 5 minutes, and then the supernatant was drawn by an aspirator. The cell pellet was loosened and suspended in 20 mL of the medium A. The spleen cell-containing suspension was filtrated using the cell strainer and changed into a 50 mL centrifugal tube. 10 mL of the spleen cell-containing suspension was centrifuged at 1500 rpm at room temperature for 5 minutes, and then the supernatant was drawn by an aspirator. The spleen cell pellet was loosened. Immediately thereafter, the loosened spleen cells were suspended in 10 mL of a serum-free animal cell culture medium (hereunder, referred to as a medium B). Then, the suspension was centrifuged at 1500 rpm at room temperature for 5 minutes, and the supernatant was drawn by an aspirator. Then, the spleen cell pellet was loosened. Next, the spleen cells was suspended in 20 mL of a cell fusion solution (hereunder, referred to as a cell fusion solution A) containing 300 mM mannitol, 0.1 mM CaCl₂, 0.2 mM MgCl₂, and 0.1 mg/mL BSA. The suspension was centrifuged at 1500 rpm at room temperature for 5 minutes, and the supernatant was drawn by an aspirator. Then, the spleen cell pellet was loosened. The spleen cells were again suspended in a small amount of the cell fusion solution A, and the suspension was filtrated using the cell strainer and changed into a 50 mL Falcon tube. The suspension was diluted with the cell fusion solution A to thereby prepare the mouse spleen cell-containing cell fusion solution A in which the final density of the spleen cells was adjusted at 0.8 × 10⁶ cells/mL. The cell fusion solution A mainly composed of 300 mM mannitol was approximately isotonic to the osmotic pressure of cells.

Next is a description of the procedure for preparing the mouse cancer cells. SP2/0 mouse cancer cells (hereunder, referred to as myeloma cells) were suspended in the medium A within a φ 15 cm Petri dish for suspension culture so that the density was always kept at 1 × 10⁶ cells/mL or lower, and subcultured in a 5% CO₂ incubator at 37°C. A day before the cell fusion, myeloma cells were suspended in the medium A so that the density of the myeloma cells was kept at 2.0 × 10⁵ cells/mL, and 40 mL of the medium A as a myeloma culture liquid was poured into the φ 15 cm Petri dish for suspension culture. The culture was performed in a total of ten Petri dishes. The myeloma culture liquid was transferred from the Petri dishes into centrifugal tubes, and centrifuged at 1000 rpm for 5 minutes. Then, the supernatant was drawn by an aspirator. The myeloma cell pellet was loosened. Immediately thereafter, 50 mL of the medium B was dispensed into the respective tubes to suspend the myeloma cells, and then collected into one tube. Then, the suspension was again centrifuged at 1000 rpm at room temperature for 5 minutes, and the supernatant was drawn by an aspirator. The myeloma cell pellet was loosened. 20 mL of the medium B was dispensed into the respective tubes to suspend the myeloma cells, and then collected into one tube. Then, the suspension was again centrifuged at 1000 rpm at room temperature for 5 minutes, and the supernatant was drawn by an aspirator. The myeloma cell pellet was loosened. Next, the myeloma cells were resuspended in 40 mL of the cell fusion solution A. The suspension was centrifuged at 1000 rpm at room temperature for 5 minutes, and the supernatant was drawn by an aspirator. Then, the myeloma cell pellet was loosened. The myeloma cells were again suspended in a small amount of the cell fusion solution A, and the suspension was filtrated using the cell strainer and changed into a 50 mL Falcon tube. The suspension was diluted with the cell fusion solution A to thereby prepare the myeloma cell-containing cell fusion solution A in which the final density was adjusted at 6.7 × 10⁶ cells/mL.
Subsequently, cell selection and cell fusion were performed by the following methods, (Method A) and (Method B), using respectively different fusion vessels.

### (Method A)

The cell fusion was performed by using the cell selection apparatus of Example 1 including the cell selection vessel in which no E2-BSA antigen was immobilized on the lower electrode on the bottom faces of the micropores. In the cell selection vessel, 600 µL of the cell fusion solution A containing the mouse spleen cells (the number of mouse spleen cells was about 0.5 million when considering the amount of loss) was introduced into the cell selection area, and the spleen cells were sufficiently settled in the cell selection area. Next, a rectangular wave AC voltage of 10 Vpp having a frequency of 3 MHz was applied between the electrodes by the first AC power supply, and thereby 0.5 million cells, approximately accounting for 50% of the about one million micropores, were immobilized in an array like arrangement where almost a single mouse spleen cell was allocated per each micropore. Subsequently, 600 µL of the cell fusion solution A containing the myeloma cells (the number of myeloma cells was about four million when considering the amount of loss of cells) was introduced into the cell selection area. In this case, since the number of myeloma cells introduced into the cell selection area was about eight times greater than the number of mouse spleen cells, it can be assumed that at least one myeloma cell was in contact onto a mouse spleen cell immobilized on a micropore. Next, the power supply was switched to the DC pulse power supply (LF101 manufactured by NEPA GENE Co., Ltd.) by the power supply-switchover mechanism, and a DC pulse voltage of 100 V having a pulse width of 30 µs was applied between the electrodes once, by which the cell fusion was performed.

### (Method B)

The cell selection and the cell fusion were performed by using the cell selection apparatus including the cell selection vessel in which the E2-BSA antigen was immobilized on the lower electrode on the bottom faces of the micropores, similar to the cell selection apparatus of Example 1. The cell selection vessel was prepared in which 600 µL of A containing the mouse spleen cells mentioned above (the number of mouse spleen cells was about 0.5 million) was introduced into the cell selection area, and the mouse spleen cells were sufficiently settled in the cell selection area. Next, a rectangular wave AC voltage of 10 Vpp having a frequency of 3 MHz was applied between the electrodes of the cell selection vessel by the first AC power supply, and thereby 0.5 million cells, approximately accounting for 50% of the about one million micropores, were immobilized in an array like arrangement where almost a single mouse spleen cell was allocated per each micropore. Next, the cell selection apparatus was left still for about two minutes to thereby effect an antigen-antibody reaction between the E2-BSA immobilized on the lower electrode on the bottom face of the micropore and the E2 antibody presented on the surface of a mouse spleen cell. Next, the power supply was switched to the second AC power supply by the power supply-switchover mechanism, and a second AC voltage (rectangular wave AC voltage of 15 Vpp having a frequency of 10 kHz) was applied to the cell selection vessel to take out mouse spleen cells which were not undergoing the antigen-antibody reaction, onto the insulating material in the vicinities of micropores, by which the cell selection was performed. Subsequently, in the fusion selection vessel, 600 µL of the cell fusion solution A containing the mouse myeloma cells (the number of mouse myeloma cells was about four million) was introduced into the cell selection area. The power supply was switched to the DC pulse power supply by the power supply-switchover mechanism and a DC pulse voltage of 100 V having a pulse width of 30 µs was applied between the electrodes once, by which the cell fusion was performed.

After the cell fusion was performed by the (Method A) or the (Method B), the cell suspension was left still as it was for 15 minutes. Then, the cell suspension inside the cell selection vessel was diluted with a HAT medium (a medium containing H: hypoxanthine, A: aminopterine, and T: thymidine components) and transferred to a 96 well plate (manufactured by Falcon). Thereafter, the fused cells were cultured in the CO₂ incubator. The HAT medium is a medium in which only the fused cells can exclusively and selectively proliferate. Six days later, the number of colonies of fused cells in each well was counted, and the refusion probability was calculated. Here, the term refusion probability means "number of colonies of fused cells generated by cell fusion/number of introduced mouse spleen cells" per each fusion vessel. Furthermore, after the number of colonies was counted, the HAT medium was all replaced. Seven days later, the presence or absence of an antibody having an E2 affinity in the culture supernatant of each well was determined by the ELISA method.

The evaluation by the ELISA method was performed in the following manner. The E2-BSA was immobilized on the ELISA plate, and then blocked by 1% skim milk. Thereafter, the culture supernatant of the fused cells was reacted to bind with an alkali phosphatase-labeled anti-mouse IgG antibody. Unreacted enzyme-labeled antibody was removed by B/F separation. Then, para-nitrophenyl phosphate (PNPP) serving as a chromogenic substrate was dispensed, and the fluorescence intensity at 405 nm was measured. A well showing an apparently higher fluorescence intensity than the background, among the thus measured wells, was determined as an E2 antibody-positive well, and the ratio of E2 antibody-positive wells was calculated. Here, the term ratio of E2 antibody-positive wells means "number of wells determined as E2 antibody positive/ number of colonies of fused cells generated by cell fusion" per each cell selection vessel. The thus calculated refusion probability and ratio of E2 antibody-positive wells per each cell selection vessel were as follows.
(Method A): The refusion probability was 16/10000 and the ratio of E2 antibody-positive wells was 0.8%.
(Method B): The refusion probability was 7/10000 and the ratio of E2 antibody-positive wells was 1.4%.

From these results, the (Method B) in which the cell selection was performed by the present invention showed a higher proportion of fused cells capable of producing E2-specific antibodies although the total number of generated fused cells was small (the refusion probability was low), as compared to the (Method A) without performing the cell selection. This indicates that the number of fused cells to be evaluated by the ELISA method (number of ELISA plates) can be reduced by performing the cell selection by taking out cells of which the specific substance on the surface of the cell is not or is weakly bound to the recognition molecule, from the micropores, under a specific force acting in a direction to take out cells from the micropores, prior to the cell fusion. Furthermore, the amount and time for the selection work to pick up cells can be remarkably reduced, and the development of monoclonal antibodies can be more efficiently performed.

### (Example 5)

Using the same cell selection apparatus as that of Example 1, the experiment was performed in the same manner as that of Example 4. A cell fusion solution A containing the mouse spleen cells and a cell fusion solution A containing mouse myeloma cells were prepared. The density of the mouse spleen cells was adjusted at 1.6 × 10⁶ cells/mL, and the density of the mouse myeloma cells was adjusted at 6.7 × 10⁶ cells/mL.
Subsequently, the cell selection and the cell fusion were performed by the following (Method C) and (Method D) respectively using different fusion vessels.

### (Method C)

The cell fusion was performed by using the cell selection apparatus of Example 1 including the cell selection vessel in which no E2-BSA antigen was immobilized on the lower electrode on the bottom faces of the micropores. In the cell selection vessel, 600 µL of the cell fusion solution A containing the mouse spleen cells mentioned above (the number of mouse spleen cells was about one million when considering the amount of loss) was introduced into the cell selection area, and the spleen cells were sufficiently settled in the cell selection area. Next, a rectangular wave AC voltage of 10 Vpp having a frequency of 3 MHz was applied between the electrodes by the first AC power supply, and thereby immobilization was done in about one million micropores in an array like arrangement where almost a single mouse spleen cell was allocated per each micropore. Subsequently, 600 µL of the cell fusion solution A containing the myeloma cells (the number of myeloma cells was about four million when considering the amount of loss of cells) was introduced into the cell selection area. In this case, since the number of myeloma cells introduced into the cell selection area was about four times grater than the number of mouse spleen cells, it can be assumed that at least one myeloma cell was in contact onto a mouse spleen cell immobilized on a micropore. Next, the power supply was switched to the DC pulse power supply (LF101 manufactured by NEPA GENE Co., Ltd.) by the power supply-switchover mechanism, and a DC pulse voltage of 100 V having a pulse width of 30 µs was applied between the electrodes once, by which the cell fusion was performed.

### (Method D)

The cell selection and the cell fusion were performed by using the cell selection apparatus including the cell selection vessel in which the E2-BSA antigen was immobilized on the lower electrode on the bottom faces of the micropores, similar to the cell selection apparatus of the (Method B) of Example 4.

The cell selection vessel was prepared in which 600 µL of the cell fusion solution A containing the mouse spleen cells mentioned above (the number of mouse spleen cells was about one million) was introduced into the cell selection area, and the mouse spleen cells were sufficiently settled in the cell selection area. Next, a rectangular wave AC voltage of 10 Vpp having a frequency of 3 MHz was applied between the electrodes of the cell selection vessel by the first AC power supply, and thereby immobilization was done in about one million micropores in an array like arrangement where almost a single mouse spleen cell was allocated per each micropore. Next, the cell selection apparatus was left still for about two minutes to thereby effect an antigen-antibody reaction between the E2-BSA immobilized on the lower electrode on the bottom face of the micropore and the E2 antibody presented on the surface of a mouse spleen cell. Next, the power supply was switched to the second AC power supply by the power supply-switchover mechanism, and a second AC voltage (rectangular wave AC voltage of 15 Vpp having a frequency of 10 kHz) was applied to the cell selection vessel to take out mouse spleen cells which were not undergoing the antigen-antibody reaction, onto the insulating material in the vicinities of micropores, by which the cell selection was performed. Subsequently, in the fusion selection vessel, a cell fusion solution A containing 0.01 % poly L-lysine (manufactured by SIGMA, hereunder abbreviated as PLL), which is one of cationic polymers, was introduced twice, and the mouse spleen cells taken out from the micropores were electrostatically immobilized on the insulating material. Next, the cell fusion solution A was introduced four times to remove excessive PLL components. Then, 600 µL of the cell fusion solution A containing the mouse myeloma cells (the number of myeloma cells was about four million) was introduced into the cell selection area. The power supply was switched to the DC pulse power supply by the power supply-switchover mechanism and a DC pulse voltage of 100 V having a pulse width of 30 µs was applied between the electrodes once, by which the cell fusion was performed.

After the cell fusion was performed by the (Method C) or the (Method D), the cell suspension was left still as it was for 15 minutes. Then, in the same manner as Example 4, the cell suspension inside the cell selection vessel was diluted with a HAT medium and transferred to a 96 well plate (manufactured by Falcon). Thereafter, the fused cells were cultured in the CO₂ incubator. Six days later, the number of colonies of fused cells in each well was counted, and the refusion probability was calculated. Furthermore, after the number of colonies was counted, the HAT medium was all replaced. Seven days later, the presence or absence of an antibody having an E2 affinity in the culture supernatant of each well was determined by the ELISA method in the same manner as that of Example 4.

The thus calculated refusion probability and ratio of E2 antibody-positive wells per each cell selection vessel were as follows.
(Method C): The refusion probability was 27/10000 and the ratio of E2 antibody-positive wells was 0.4%.
(Method D): The refusion probability was 2/10000 and the ratio of E2 antibody-positive wells was 3.8%.

From these results, the (Method D) in which the cell selection was performed by the present invention and also the spleen cells taken out from the micropores were electrostatically immobilized on the insulating material by PLL to thereby prevent the reimmobilization onto the micropores, showed a much higher proportion of fused cells capable of producing E2-specific antibodies than that of Example 4 although the total number of generated fused cells was small (the refusion probability was low), as compared to the (Method C) without performing the cell selection. This indicates that the number of fused cells to be evaluated by the ELISA method (number of ELISA plates) can be much reduced by performing the cell selection by taking out cells of which the specific substance on the surface of the cell is not or is weakly bound to the recognition molecule, from the micropores, under a specific force acting in a direction to take out cells from the micropores, and subsequently after the cell selection, capturing the cells taken out from the micropores on a different portion other than the micropores, to thereby prevent the reimmobilization onto the micropores in advance prior to the cell fusion. Furthermore, the amount and time for the selection work to pick up cells can be remarkably reduced, and the development of monoclonal antibodies can be more efficiently performed.

### (Comparative Example 2)

The mouse spleen B cells selected in Comparative Example 1 were subjected to cell fusion by a normal electrofusion method. The electrodes for performing the electro cell fusion were 1 mm-spaced gold wire electrodes (MS gold wire electrodes manufactured by NEPA GENE Co., Ltd.) connected with the cell-fusion power supply (LF101 manufactured by NEPA GENE Co., Ltd.).
The cells used herein were mouse spleen B cells and mouse myeloma cells selected in Comparative Example 1. The mouse antibody-producing cells B and the mouse myeloma cells were mixed at a ratio of 4:1. Both types of cells were suspended in a 300 mM mannitol aqueous solution to adjust the cell suspension at a density of 1.7 × 10⁷ cells/mL. To the suspension containing both types of cells were added 0.1 mM calcium chloride and 0.1 mM magnesium chloride so as to promote the regeneration of cell membranes in the cell fusion.

40 µL of the cell suspension (the number of mouse antibody-producing cells was about 0.6 million and the number of mouse myeloma cells was about 0.15 million) was injected between the electrodes. A sine wave AC voltage of 20 Vpp having a frequency of 3 MHz was applied between the electrodes by using the cell-fusion power supply. After ensuring the formation of a pearl chain of cells, a DC pulse voltage of 200 V having a pulse width of 30 µs was applied for performing the cell fusion. After leaving it for 10 minutes, the cell suspension inside the cell selection vessel was poured into a HAT medium. The HAT medium containing the cell suspension was placed in a CO₂ incubator to perform cell culture. Six days later, the number of fused cells was counted. As a result, no fused cell was able to be found. This can be considered because it was substantially impossible to generate fused cells because the number of mouse spleen B cells left by the selection was small, and the number was further reduced due to the amount of loss during the transfer of mouse spleen B cells into the electrodes for the cell fusion, due to the density adjustment and the centrifugation of the cell suspension, and in addition the refusion probability of a normal electrofusion method is extremely low at about 0.2/10000.

### INDUSTRIAL APPLICABILITY

According to the cell selection apparatus and the cell selection method of the present invention, it is possible to immobilize a single cell per each micropore under the positive dielectrophoretic force from the first AC power supply. Moreover, each cell can be taken out from the micropore under the specific force acting in a direction to take out the cell from the micropores so as to be individually selected, making it possible to select a mass of cells on the surfaces of which a specific substance strongly bound to a recognition molecule is presented, all at once easily in a short period of time. Accordingly, the present invention is industrially applicable in the fields of drug manufacture and clinical diagnosis.

### BRIEF DESCRIPTION OF THE REFERENCE SYMBOLS

1: Cell selection area
2: Cell solution
3: Conductive line
4: Power supply
5: First AC power supply
6: Second AC power supply
7: Power supply-switchover mechanism
8: Insulating material
9: Micropores
10: Positive dielectrophoretic force
11: Site where electric force lines are concentrated
12: Electric force lines
13: Cell selection vessel
14: Upper electrode
15: Lower electrode
16: Spacer
17: Cell of which the specific substance on the surface of the cell is not or is weakly bound to the recognition molecule
18: Cell of which the specific substance on the surface of the cell is bound to the recognition molecule
19: Inlet port
20: Outlet port
21: Negative dielectrophoretic force
22: Binding force between the specific substance on the surface of the cell and the recognition molecule
23: ITO
24: Pyrex (registered trademark) glass
25: Resist
26: Exposure photomask
27: Exposure
28: Micropored insulating material-integrated lower electrode
29: Inlet channel
30: Outlet channel
31: Peak voltage
32: Developing solution
33: Specific substance
34: Recognition molecule
35: DC pulse power supply
36: Gravitational force
37: Magnetic force
38: Magnetic body
39: Fluid delivery force
40: Antibody
41: Antigen
42: Mouse spleen cell
43: Mouse cancer cell
44: Anti-IgG antibody
45: Magnetic fine particles
46: Cationic polymer
47: IgG-type antibody among the entire population of antibodies on cell surfaces of mouse spleen cells
48: Mouse spleen cell presenting a small proportion of IgG-type antibodies on its surface, among the entire population of the mouse spleen cells
49: Mouse spleen cell presenting a large proportion of IgG-type antibodies on its surface, among the entire population of the mouse spleen cells 50: Cell

## Claims

1. A cell selection apparatus comprising:
a cell selection vessel which comprises a pair of electrodes formed from oppositely disposed conductive members and a sheet-like insulating material disposed between said pair of electrodes via a sheet-like spacer and having a plurality of micropores piercing in the direction of said oppositely disposed electrodes, with said insulating material being disposed on a surface of either one of said electrodes, and a recognition molecule bindable to a specific substance being disposed on a bottom face of said micropore; and
a power supply for applying a voltage to said pair of electrodes, wherein
said power supply comprises a cell-immobilization power supply and a cell-taking power supply.

2. The cell selection apparatus according to claim 1, wherein, in said power supply, said cell-immobilization power supply comprises a first AC power supply for applying a first AC voltage, said cell-taking power supply comprises a second AC power supply for applying a second AC voltage, and said power supply comprises a power supply-switchover mechanism for switching over between said first AC power supply and said second AC power supply.

3. The cell selection apparatus according to claim 1, wherein said power supply comprises a cell-fusion power supply in addition to said cell-immobilization power supply and said cell-taking power supply.

4. The cell selection apparatus according to claim 3, wherein, in said power supply, said cell-immobilization power supply comprises a first AC power supply for applying a first AC voltage, said cell-taking power supply comprises a second AC power supply for applying a second AC voltage, said cell-fusion power supply comprises a DC pulse power supply for applying a DC pulse voltage, and a power supply-switchover mechanism which picks up any one or two of power supply selected from the group consisting of said first AC power supply, said second AC power supply, and said DC pulse power supply, and is connected to the picked up one.

5. The cell selection apparatus according to claim 2 or claim 4, wherein an AC frequency of said first AC power supply is 30 kHz or higher, and an AC frequency of said second AC power supply is lower than 20 kHz.

6. The cell selection apparatus according to any one of claims 1 through 5, wherein said recognition molecule bindable to the specific substance is an antigen.

7. The cell selection apparatus according to any one of claims 1 through 6, wherein a cationic polymer is provided on a surface of said electrode which faces a cell selection area serving as a space for selecting cells and being formed between said pair of electrodes of said cell selection vessel via said spacer, and/or a surface of said micropored plate-like insulating material disposed on the electrode on the side of the insulating material, of said cell selection vessel.

8. The cell selection apparatus according to claim 7, wherein said cationic polymer is a polylysine, a polylysine derivative, or an amino group-containing polymer.

9. A cell selection method using the cell selection apparatus according to any one of claims 1 through 8, comprising:
introducing cells into said cell selection area;
immobilizing said cells in said micropores by applying said first AC voltage from said first AC power supply;
effecting a binding reaction between said specific substance on a surface of said cell and said recognition molecule disposed on a bottom face of said micropore; and
thereafter, taking out a cell of which the specific substance on the surface of said cell is not or is weakly bound to said recognition molecule, among said cells, from said micropore, under a specific force acting in a direction to take out said cell from said micropore.

10. A cell selection method using the cell selection apparatus according to any one of claims 1 through 8, comprising:
introducing cells into said cell selection area;
immobilizing said cells in said micropores by applying said first AC voltage from said first AC power supply;
effecting a binding reaction between said specific substance on a surface of said cell and said recognition molecule disposed on a bottom face of said micropore; and
thereafter, leaving a cell of which the specific substance on the surface of said cell is strongly bound to said recognition molecule, among said cells, behind in the micropore, under a specific force acting in a direction to take out said cell from said micropore.

11. The cell selection method using the cell selection apparatus according to any one of claims 3 through 8, comprising:
introducing cells into said cell selection area;
immobilizing said cells in said micropores by applying said first AC voltage from said first AC power supply;
effecting a binding reaction between said specific substance on a surface of said first cell and said recognition molecule;
thereafter, taking out a cell of which the specific substance on the surface of said cell is not or is weakly bound to said recognition molecule, among said first cells, from said micropore, under a specific force acting in a direction to take out said cell from said micropore;
subsequently introducing second cells into said cell selection area to bring these second cells into contact with said first cells remaining in said micropores;
switching over to said DC pulse power supply, by said power supply-switchover mechanism;
applying said DC pulse voltage to said cells; and
effecting cell fusion between said first cell remaining in said micropore and said second cell contacting thereto.

12. The cell selection method according to any one of claims 9 through 11, wherein said specific force is a gravitational force acting in a direction to take out said cell from said micropore.

13. The cell selection method according to any one of claims 9 through 11, wherein said specific force is a magnetic force acting in a direction to take out said cell from said micropore.

14. The cell selection method according to any one of claims 9 through 11, wherein said specific force is a fluid delivery force of a solution to be introduced into said cell selection vessel, acting in a direction to take out said cell from said micropore.

15. The cell selection method according to any one of claims 9 through 11, wherein said specific force is a dielectrophoretic force acting in a direction to take out said cell from said micropore, through an application of said second AC voltage from said second AC power supply which has been switched over to by said power supply-switchover mechanism.

16. The cell selection method according to any one of claims 9 through 11, wherein said specific force is a combination of two or more of said specific forces according to any one of claims 12 through 15.

17. The cell selection method according to claim 14 or claim 16, wherein
said specific force is a fluid delivery force acting in a direction to take out said cell from said micropore, and
cells are selected into cells of which said specific substance on the surface of said cell is not or is weakly bound to said recognition molecule, and cells of which the specific substance on the surface of said cell is strongly bound to said recognition molecule, by changing a strength of said fluid delivery force through variation of a rate of the fluid delivery velocity of a solution to be introduced into said cell selection vessel.

18. The cell selection method according to claim 15 or claim 16, wherein
said specific force is a dielectrophoretic force acting in a direction to take out said cell from said micropore, and
cells are selected into cells of which said specific substance on the surface of said cell is not or is weakly bound to said recognition molecule, and cells of which the specific substance on the surface of said cell is strongly bound to said recognition molecule, by changing a strength of said dielectrophoretic force through variation of the magnitude and/or a frequency of said second AC voltage from said second AC power supply.

19. The cell selection method according to any one of claims 9 through 18, comprising:
capturing cells taken out from said micropores, onto said surface of said electrode on a side of said cell selection area, and/or said surface of said micropored plate-like insulating material disposed on said electrode on a side of the insulating material, where said cationic polymer has been provided, according to claim 7 or 8.

20. The cell selection method according to any one of claims 11 through 19, comprising:
immobilizing said recognition molecule which can recognize said specific substance of said first cell, onto a surface of said second cell; and
binding said first cell to said second cell through a bond between said specific substance and said recognition molecule.

21. The cell selection method according to claim 20, comprising:
fusing said first cell and said second cell by applying said DC pulse voltage while applying said specific force acting in a direction to take out said cell from said micropore.

22. A cell selection apparatus comprising:
oppositely facing first and second electrodes, and
a power supply apparatus for applying an AC voltage to these electrodes,
wherein
an insulating material having micropores is disposed on a surface on said second electrode side of said first electrode,
a portion of said micropores but for an electrode exposure site in contact with said micropores is covered with the insulating material,
said first and second electrodes are disposed so that said insulating material and the second electrode are separated,
a recognition molecule is disposed on said electrode exposure site, and
said power supply apparatus applies two types of AC voltages having different frequencies to the first and second electrodes.
